# EUROPEAN PATENT APPLICATION

(11) **EP 1 231 209 A1**
(43) Date of publication of application: **14.08.2002**
(21) Application number: 01130083.7
(22) Date of filing: 18.12.2001
(51) Int. Cl.: C07D 295/20, C07D 209/26, C07D 487/04, A61K 31/535, A61P 29/00

(54) **Prodrugs of non-steroidal anti-inflammatory and carboxylic acid containing compound**

(30) Priority: 19.12.2000 US 256634 P
(71) Applicant: Specialized Pharmaceutical Research Ltd. Co., Amman (JO)
(72) Inventor: Jilani, Jamal A., Amman 11195 (JO)
(74) Representative: Leissler-Gerstl, Gabriele

(57) **Abstract**

Compounds of the formula: RC(O)O-spacer-OC(O)R' are claimed, wherein (i) RC(O)- is the acyl residue of an NSAID or other pharmaceutically active agent bearing a carboxylic acid function, (ii) spacer is Cₙ alkyl, (iii) n is from 1 to 6, and (iv) R' is substituted or unsubstituted heteroaryl or heterocycle, and pharmaceutical compositions thereof. The compounds are prodrugs of NSAIDS and can be used to treat inflammation.

## Description

### FIELD OF THE INVENTION

The present invention concerns novel prodrugs of non-steroidal anti-inflammatory drugs ("NSAIDs") and other pharmaceutical compounds that contain a carboxylic acid function, and especially to morpholino-carbonyloxyethyl esters of such NSAIDS and other drugs. The prodrugs of the present invention are especially useful for treating inflammation and other disorders that respond to NSAIDs. The invention also concerns processes for preparing such prodrugs, and to pharmaceutical compositions containing them.

### BACKGROUND OF THE INVENTION

Nonsteroidal anti-inflammatory drugs (NSAIDs) are prescribed extensively throughout the world, and are used principally to treat pain, fever and inflammation as a result of acute injuries, rheumatoid arthritis, and osteoarthritis. Loeb DS, Talley NJ, Ahlquist DA, *Gastroenterology* 1992;102(6):1899-905; Zeidler H., *J. Rheumatol.* 1991;(Suppl 28):2-5.

The major physiological effect of all NSAIDs is to decrease the synthesis of prostaglandins by inhibiting cyclooxygenase (COX). The COX enzyme catalyzes the formation of prostaglandin from arachidonic acid. Prostaglandins are a natural target for treating inflammatory disorders because they have been shown to contribute to inflammatory responses. However, they also perform several other vital functions, by enhancing renal blood flow and protecting the cellular morphology of gastrointestinal mucosa.

NSAIDs inhibit cyclooxygenase via several different biological pathways. For example, aspirin suppresses COX activity by irreversibly acetylating Serine-530 of the COX enzyme and thereby blocking access of arachidonic acid to the active site. Other NSAIDs, such as indomethacin, are allosteric inhibitors of COX, and form a tight, slowly dissociable complex with COX that induces an inhibitory conformational change. Ibuprofen and piroxicam, on the other hand, compete with arachidonic acid for the active enzymatic binding site of COX.

NSAIDs with which people are most familiar include aspirin, indomethacin, sulindac, ibuprofen, and piroxicam. For convenience, however, the drugs are generally broken down into the following chemical classes:
- p-Aminophenols such as Acetaminophen,
- Salicylates such as Aspirin,
- Pyrazolidinediones such as Phenylbutazone,
- Arylacetic acids such as Indomethacin,
- Arylpropionic acids such as Ibuprofen,
- Fenamic Acids such as Mefenamic Acid, and
- Oxicams such as Piroxicam.

A significant percentage of patients taking NSAIDs report some type of adverse gastrointestinal effect, ranging from dyspepsia to generalized abdominal discomfort. In a minority of users, severe complications, including gastric and duodenal ulcerations, gastrointestinal bleeding or perforation, and mucosal injury to either the small or large intestine, develop. The organs most commonly affected by ulceration in NSAID users are the stomach (12% to 30%) and the duodenum (2% to 19%), though there is some risk of injury to the esophagus, small bowel, and colon. Geis GS, Stead H, Wallemark CB, *J. Rheumatol.* 1991;(Suppl 28):11-4.

In the presence of gastric acid, weak-acid NSAIDs such as indomethacin and ketoprofen diffuse freely across the gastric mucosal barrier and become ionized and sequestered in the mucosal cells, an occurrence that leads to cytotoxicity. NSAIDs cause local damage through the inhibition of cyclooxygenase, and may also exert a direct toxic effect upon the mucosal cells. Some investigators have postulated that when NSAIDs are concentrated in the mucosa, they may alter local immune responses that direct leukocytes against the gastric mucosa. Person, SP, *Postgraduate Medicine* 1996, 100(5): 1-8. Regardless of the precise biological mechanism through which localized NSAIDs damage the gastric mucosa, it is generally recognized that the localization of NSAIDs in the gastric mucosa should be reduced if at all possible.

This has been demonstrated for salsalate, a nonacetylated salisalicylate NSAID. Salsalate is insoluble at normal acidic gastric pH and therefore does not inhibit gastric mucosal prostaglandin synthesis appreciably. As a result, topical gastric injury is generally less than with enteric-coated acetylsalicylic acid (ASA), despite equivalent serum salicylate concentrations. These results should be contrasted, however, with several studies in which the relative risk of dose-related injury with use of indomethacin, naproxen, tolmetin sodium, and meclofenamate sodium was shown to be significantly greater than that with ibuprofen. Griffin MR, Piper JM, Daugherty JR, *et al., Ann. Intern. Med.* 1991;114(4):257-63; Gabriel SE, Jaakkimainen L, Bombardier C., *Ann. Intern. Med.* 1991;115(10):787-96.

Recently, the use of prodrugs to reduce adverse effects has provided some optimism. For example, prodrugs such as nabumetone and etodolac confer added gastric mucosal protection by not significantly inhibiting gastric prostaglandin synthesis. Postmarketing surveillance data and short-term endoscopic studies indicate that the incidence of gastroduodenal erosive injury is lower (<1%) with both of these agents. Cummings DM, Amadio P Jr., *Am. Fam. Physician* 1994;49(5):1197-202. However, the prototype prodrug sulindac, which was designed to avoid topical proximal gastrointestinal tract reactions through its hepatic metabolism to an active form, appears to offer little additional protective advantage.

Esterified NSAID prodrugs, which are reportedly enzymatically degraded *in vivo* to an active carboxylic acid form, are reported in US Patent No. 4,542,158 issued Sept. 17, 1985, US Patent No. 4,851,426 issued July 25, 1989, and US Patent No. 5,998,465 issued Dec. 7,1999. US '158 discloses 1-(alkoxy or aroxy)carbonyloxyalkyl esters of diflunisal. The diflusinal is esterified by a moiety of the general formula -C(R¹)HOC(O))R, wherein R¹ is hydrogen, lower alkyl, lower cycloalkyl, or aryl, and R is lower alkyl, lower cycloalkyl, or aryl.

US '426 discloses prodrugs of NSAIDs of the general formula RC(O)OCH(CH₃)OC(O))CH₂CH₃. Examples of NSAIDS that can be converted into the prodrugs include aspirin, indomethacin, naproxen, ibuprofen, sulindac, diflusinal, ketoprofen, mefenamic acid, tolmetin, diclofenac, and flufenamic acid. The prodrugs are prepared by esterifying the parent NSAID with a compound of formula XCH(CH₃)OC(O))CH₂CH₃, wherein X is bromine or chlorine.

US '465 also discloses prodrugs of NSAIDs, wherein the NSAID is linked to an esterifying agent through an ester bond. The esterifying agent is preferably a benzopyran, which has been linked by a condensation reaction with an NSAID comprising a carboxylic acid function.

Despite these advances in NSAID delivery, there remains a need to develop NSAID prodrugs that are less harmful to the patients to whom they are administered, and that minimize gastrointestinal tract side effects to such patients. It would be advantageous in the delivery of NSAID's to mask the carboxyl function of the drug to prevent localization of the drug in the gastric mucosa. Such a process would also be advantageous in the delivery of drugs other than NSAID's, especially for those drugs which are associated with gastrointestinal disorders, because of the ability of prodrugs produced by the process to avoid sequestration in the gastric mucosa.

Thus, it is an object of the invention to minimize the gastrointestinal tract side effects associated with orally administered NSAIDs, and to prevent the localization of NSAIDs in the gastric mucosa of affected patients.

It is another object of the present invention to provide prodrugs of NSAIDs that effectively treat inflammation and inflammatory disorders, and that treat inflammation and inflammatory disorders at least as effectively as the parent compound.

Still another object of the present invention is to provide prodrugs of NSAIDs that are as bioavailable *in vivo* as the parent compound.

A further object of the invention is to optimize the physicochemical properties of NSAIDs when delivered topically or ophthalmicly

Yet another object of the present invention is to provide methods of treating inflammation and inflammatory disorders using the NSAID prodrugs of the present invention.

Another object of the invention is to provide novel chemical entities from which the NSAID prodrugs of the present invention can be synthesized and which, when cleaved from the parent NSAID *in vivo,* are cleared from the body.

A still further object of the invention is to provide prodrugs of pharmaceutical compounds other than NSA-IDs.

### SUMMARY OF THE INVENTION

The present invention provides novel prodrugs of NSAIDs and other pharmaceutically active agents which, in their native form, comprise one or more carboxylic acid functions. The prodrugs are less toxic to the gastrointestinal system than the native drug and, when administered orally, are absorbed from the GUT into the blood stream where they liberate their corresponding parent drugs, or exhibit independent pharmacological activity of themselves. The invented prodrugs typically exhibit greater ability to penetrate through skin tissues than the corresponding parent compounds. Moreover, when administered topically, the invented prodrugs hydrolyze upon absorption by the skin tissue to yield the parent drugs, or exhibit independent pharmacological activity. The present invention also novel prodrugs of NSAIDS and pharmaceutical compounds other than NSAIDs which, in their native form, comprise one or more carboxylic acid functions.

*In vivo,* the invented prodrugs typically are selectively hydrolyzed by plasma enzymes to yield the parent NSAIDs or other pharmaceutical compound, but are otherwise stable against chemical hydrolysis. For example, the morpholinecarbonyloxyethyl ester of diclofenac exhibits a half-life of 21 minutes in plasma at physiologic pH. In contrast, the ester exhibits a half life of 8 hours in a 1.0 pH solution, and 47 hours in a 7.4 pH solution. Similarly, the morpholinecarbonyloxyethyl ester of mefenamic acid exhibits a half-life of 20 minutes in plasma, 7.6 hours in a 1.0 pH solution, and 66 hrs in a 7.4 pH solution.

Thus, in one embodiment the invention provides prodrugs represented by the formula: RC(O)O-spacer-OC(O)R', wherein:
a. RC(O)- is the acyl residue of an NSAID or other pharmaceutically active agent bearing a carboxylic acid function,
b. spacer is Cₙ alkyl,
c. n is 1, 2, 3, 4, 5, or 6, and
d. R' is substituted or unsubstituted heteroaryl or heterocycle.

In a particularly preferred embodiment the prodrugs are represented by the structure: 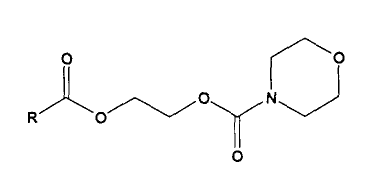 wherein RC(O)- is the acyl residue of a NSAID or other pharmaceutically active agent that bears a carboxylic acid function.

The prodrugs are typically prepared by esterifying NSAIDs and other drugs that bear a carboxylic acid function with a compound of the formula X-spacer-OC(O)R', wherein X is a leaving group, spacer is C₁₋₆ alkyl, and R' is substituted or unsubstituted heteroaryl or heterocycle. In a particularly preferred embodiment the NSAID prodrugs are obtained by esterifying the NSAID carboxylic acid function with N-[(2-haloethyloxy)carbonyl]morpholine, which is represented by the following structure when X is halogen, preferably bromine, chlorine, or iodine:

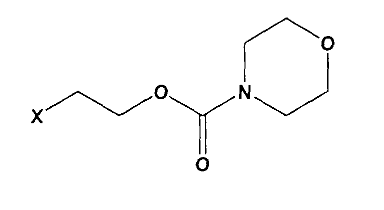

In another embodiment, the invention provides compounds with which the carboxylate group of carboxyl containing drugs can be esterified. Thus, in another embodiment the invention provides novel compounds of the formula X-spacer-OC(O)R', wherein X is a leaving group, and spacer and R' are defined above. A preferred such compound is N-[(2-haloethyloxy)carbonyl]morpholine, and especially N-[(2-bromoethyloxy)carbonyl]morpholine.

In still another embodiment the invention provides a method of treating a disease, preferably inflammation or an inflammatory disorder, comprising administering to a subject diagnosed as suffering from the disease an effective treatment amount of a compound of the formula: RC(O)O-spacer-OC(O)R', wherein: RC(O)- is the acyl residue of an NSAID or other pharmaceutically active agent bearing a carboxylic acid function, spacer is Cₙ alkyl, n is 1-6, and R' is substituted or unsubstituted heteroaryl or heterocycle. Any mode of administration is suitable, but topical, opthalmical, and oral modes of administration are especially preferred. RC(O)O-spacer-OC(O)R' is preferably an NSAID esterified by N-[(2-haloethyloxy)carbonylmorpholine.

Non-limiting examples of parent NSDAIDs suitable for prodrug modification according to the present invention include diclofenac, indomethacin, ketorolac, ketoprofen, ibuprofen, naproxen, diflunisal, mefenamic acid, ioxoprofen, tolfenamic acid, indoprofen, pirprofen, fenoprofen, zaltoprofen, sulindac, tolmetin, suprofen, flubiprofen, pranoprofen, niflumic acid, flufenamic acid, zomopirac, bromfenac, fenclofenac, alcofenac, orpanoxin, etodolic acid, fenclozic acid, amfenac, emfenamic acid, benoxaprofen, flunoxaprofen, carprofen, isofezolac, aceclofenac, fenbufen, fenclorac, meclofenamate, clindac, among others.

### DETAILED DESCRIPTION OF THE INVENTION

### Discussion

As mentioned above, the inventors have discovered a novel class of compounds with which NSAIDs and other drugs that possess a carboxylic acid function are esterified to provide prodrugs having improved pharmacological properties. Thus, in one embodiment the invention provides prodrugs represented by the formula: RC(O)O-spacer-OC(O)R', wherein:
a. RC(O)- is the acyl residue of an NSAID or other pharmaceutically active agent bearing a carboxylic acid function,
b. spacer is Cₙ alkyl,
c. n is 1, 2, 3, 4, 5, or 6, and
d. R' is substituted or unsubstituted heteroaryl or heterocycle.

In a particularly preferred embodiment the prodrugs are represented by the structure: 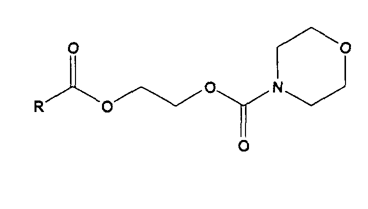 wherein RC(O)- is the acyl residue of a NSAID or other pharmaceutically active agent that bears a carboxylic acid function.

Thus, the invention provides:
1. Prodrugs of carboxylic acid containing NSAIDs and other pharmaceutical agents of a defined formula, and pharmaceutically acceptable salts thereof;
2. Novel esterifying compounds with which NSADs and other pharmaceutical agents that contain a carboxylic acid function can be converted to esterified prodrugs;
3. Methods of making prodrugs of carboxylic acid containing NSAIDs and other pharmaceutical agents with the novel esterifying compounds of this invention;
4. Pharmaceutical formulations that contain the prodrugs of the present invention, especially oral, topical, and ophthalmic formulations;
5. Methods of using the prodrugs of the present invention in the treatment of inflammation and inflammatory disorders, and other disease states;
6. Methods of reducing the gastrointestinal side effects associated with NSAIDs and other pharmaceutical agents that contain a carboxylic acid function; and
7. Methods of improving the topical delivery profile of NSAIDs and other pharmaceutical agents that contain a carboxylic acid function.
8. Methods of manufacturing medicaments comprising the prodrugs for the treatment of disease states, including inflammation and inflammatory disorders.

### Definitions and Use of Terms

In the context of the present specification the term "prodrug" denotes a derivative of a known and proven NSAID or other pharmaceutical agent having a carboxylic acid function.

Halo is fluoro, chloro, bromo, or iodo.

The term alkyl, as used herein, unless otherwise specified, refers to a saturated straight, branched, or cyclic, primary, secondary, or tertiary hydrocarbon of C₁ to C₁₀, and specifically includes methyl, ethyl, propyl, isopropyl, cyclopropyl, butyl, isobutyl, *t*-butyl, pentyl, cyclopentyl, isopentyl, neopentyl, hexyl, isohexyl, cyclohexyl, cyclohexylmethyl, 3-methylpentyl, 2,2-dimethylbutyl, and 2,3-dimethylbutyl. Although the invention encompasses both substituted and unsubstituted alkyl, unless specifically referred to as "unsubstituted," the term alkyl includes substituted alkyl. Moieties with which the alkyl group can be substituted are selected from the group consisting of hydroxyl, amino, alkylamino, arylamino, alkoxy, aryloxy, aryl, heterocycle, halo, carboxy, acyl, acyloxy, amido, nitro, cyano, sulfonic acid, sulfate, phosphonic acid, phosphate, or phosphonate, either unprotected, or protected as necessary, as known to those skilled in the art, for example, as taught in Greene, *et al.,* Protective Groups in Organic Synthesis, John Wiley and Sons, Second Edition, 1991, hereby incorporated by reference. Examples of substituted alkyl groups include trifluoromethyl and hydroxymethyl.

The term lower alkyl, as used herein, and unless otherwise specified, refers to a C₁ to C₄ saturated straight, branched, or if appropriate, a cyclic (for example, cyclopropyl) alkyl group, including both substituted and unsubstituted forms. Unless otherwise specifically stated in this application, when alkyl is a suitable moiety, lower alkyl is preferred. Similarly, when alkyl or lower alkyl is a suitable moiety, unsubstituted alkyl or lower alkyl is preferred.

The term "-(CH₂)ₙ-" represents a saturated alkylidene radical of straight chain configuration. The term "n" can be any whole integer, including 0, 1, 2, 3, 4, 5, 6, 7, 8, 9, or 10. The moiety "-(CH₂)ₙ-" thus represents a bond (*i.e.,* when n=0), methylene, 1,2-ethanediyl or 1,3-propanediyl, etc.

The term aryl, as used herein, and unless otherwise specified, refers to phenyl, biphenyl, or naphthyl, and preferably phenyl. The aryl group can be optionally substituted with one or more moieties selected from the group consisting of hydroxyl, acyl, amino, halo, carboxy, carboxamido, carboalkoxy, alkylamino, alkoxy, aryloxy, nitro, cyano, sulfonic acid, sulfate, phosphonic acid, phosphate, or phosphonate, either unprotected, or protected as necessary, as known to those skilled in the art, for example, as taught in *Greene, et al.,* "Protective Groups in Organic Synthesis," John Wiley and Sons, Second Edition, 1991.

The term heteroaryl or heteroaromatic, as used herein, refers to an aromatic or unsaturated cyclic moiety that includes at least one sulfur, oxygen, nitrogen, or phosphorus in the aromatic ring. Nonlimiting examples are furyl, pyridyl, pyrimidyl, thienyl, isothiazolyl, imidazolyl, tetrazolyl, pyrazinyl, benzofuranyl, benzothiophenyl, quinolyl, isoquinolyl, benzothienyl, isobenzofuryl, pyrazolyl, indolyl, isoindolyl, benzimidazolyl, purinyl, carbazolyl, oxazolyl, thiazolyl, isothiazolyl, 1,2,4-thiadiazolyl, isooxazolyl, pyrrolyl, quinazolinyl, pyridazinyl, pyrazinyl, cinnolinyl, phthalazinyl, quinoxalinyl, xanthinyl, hypoxanthinyl, and pteridinyl. Functional oxygen and nitrogen groups on the heteroaryl group can be protected as necessary or desired. Suitable protecting groups are well known to those skilled in the art, and include trimethylsilyl, dimethylhexylsilyl, *t*-butyldimethylsilyl, and *t*-butyldiphenylsilyl, trityl or substituted trityl, alkyl groups, acycl groups such as acetyl and propionyl, methanesulfonyl, and p-toluenelsulfonyl. The heteroaryl or heteroaromatic group can be optionally substituted with one or more moieties selected from the group consisting of hydroxyl, acyl, amino, halo, alkylamino, alkoxy, aryloxy, nitro, cyano, sulfonic acid, sulfate, phosphonic acid, phosphate, or phosphonate, either unprotected, or protected as necessary, as known to those skilled in the art, for example, as taught in *Greene, et al.,* "Protective Groups in Organic Synthesis," John Wiley and Sons, Second Edition, 1991.

The term heterocyclic refers to a saturated nonaromatic cyclic group which may be substituted, and wherein there is at least one heteroatom, such as oxygen, sulfur, nitrogen, or phosphorus in the ring. The heterocyclic group is preferably linked through a carbon atom to the N-substituted dithiocarbamate. The heterocyclic group can be substituted in the same manner as described above for the heteroaryl group.

The term alkoxy, as used herein, and unless otherwise specified, refers to a moiety of the structure -O-alkyl, wherein alkyl is as defined above.

The term amino, as used herein, refers to a moiety represented by the structure -NR₂, and includes primary amines, and secondary, and tertiary amines substituted byalkyl, aryl, heterocycle, acyl, and sulfinylalkyl. Thus, R₂ may represent two hydrogens, two alkyl moieties, or one hydrogen and one alkyl moiety.

The term pharmaceutically acceptable salts or complexes refers to salts or complexes that retain the desired biological activity of the compounds of the present invention and exhibit minimal undesired toxicological effects. Nonlimiting examples of such salts are (a) acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acid, naphthalenedisulfonic acid, and polygalcturonic acid; (b) base addition salts formed with metal cations such as zinc, calcium, bismuth, barium, magnesium, aluminum, copper, cobalt, nickel, cadmium, sodium, potassium, and the like, or with a cation formed from ammonia, N,N-dibenzylethylenediamine, D-glucosamine, tetraethylammonium, or ethylenediamine; or (c) combinations of (a) and (b); *e.g.*, a zinc tannate salt or the like. Also included in this definition are pharmaceutically acceptable quaternary salts known by those skilled in the art, which specifically include the quaternary ammonium salt of the formula -NR⁺A⁻, wherein R is as defined above and A is a counterion, including chloride, bromide, iodide, -O-alkyl, toluenesulfonate, methylsulfonate, sulfonate, phosphate, or carboxylate (such as benzoate, succinate, acetate, glycolate, maleate, malate, citrate, tartrate, ascorbate, benzoate, cinnamoate, mandeloate, benzyloate, and diphenylacetate).

Nonlimiting examples of inflammatory disorders include rheumatoid and osteoarthritis, asthma, dermatitis, psoriasis, cystic fibrosis, post transplantation acute and chronic solid organ rejection, multiple sclerosis, atherosclerosis, post-angioplasty restenosis, and angina.

### Esterifying Compounds

Esterifying compounds which are useful in the preparation of prodrugs according to the present invention can be represented generally by the formula: X-spacer-OC(O)R', wherein:
a. X is a leaving group,
b. spacer is Cₙ alkyl,
c. n is 1, 2, 3, 4, 5, or 6, and
d. R' is substituted or unsubstituted heteroaryl or heterocycle.

The term "a leaving group" refers to a class of compounds with which those of skill in the art of organic chemistry are familiar. As used herein, the term "leaving group" refers to the class of compounds that mediates nucleophilic substitution on a substrate. The substrate to which the leaving group is attached can thus be attacked by a nucleophilic reagent such as hydroxide, alkoxide, cyanide, ammonia or water. The leaving group preferably is capable of mediating nucleophilic attack by a carboxyl function. Leaving groups represented by X generally include the halides (i.e. fluorine, chlorine, bromine, and iodine), and sulfonates such as tosylate.

Spacer is preferably -(CH₂)ₙ-. Moreover, n is preferably 1-4. Most preferably, spacer is ethylene.

In a preferred embodiment R' is NR¹R², wherein R¹ and R² are C₂₋₄ alkyl or heteroalkyl that join to form a 5-7 membered heterocyclic or heteroaromatic ring, substituted or unsubstituted. In a particularly preferred embodiment R¹ and R² combine to form morpholine.

### Parent Compounds

As mentioned above, prodrugs of the present invention can be prepared from any drug or pharmaceutically active agent that possesses a carboxylate function. The term drug or pharmaceutically active agent refers to any chemical compound that exhibits a beneficial *in vivo* biological effect when administered to a mammalian species. The term "carboxylate" refers to a moiety represented by the structure -COOH, and also includes carboxylate salts.

NSAIDs that possess carboxylate functions are particularly preferred as the parent drug. An NSAID is defined as any compound that decreases the synthesis of a prostaglandin by inhibiting cyclooxygenase (COX). Inhibition can be by any available pathway, including by irreversibly acetylating Serine-530 of the COX enzyme and thereby blocking access of arachidonic acid to the active site (as in aspirin), allosteric inhibition (such as indomethacin), and competitive inhibition for the active enzymatic binding site of COX (as in ibuprofen and piroxicam).

NSAIDs having a carboxylate function can generally be broken down into the following chemical classes:
- Salicylates such as Aspirin,
- Arylacetic acids such as Indomethacin,
- Arylpropionic acids such as Ibuprofen, and
- Fenamic Acids such as Mefenamic Acid.

Salicylates: The salicylate class refers to NSAIDs that are derived from salicylic acid, a natural product present in the bark of willow and poplar trees. Aspirin, the acetyl ester of salicylic acid, is the most common salicycylate used as an NSDAID. Many congeners of aspirin have been developed principally to overcome gastrointestinal problems inherent in the class, including salsalate, a dimer of salicylic acid linked through the ester, salicylamide, and various salts of salicylic acid. Diflunisal is also considered a salicylate despite its major substitutions on the salicylate molecule.

Salicylic acid, aspirin, and salicylamide can be represented by the following chemical structures:

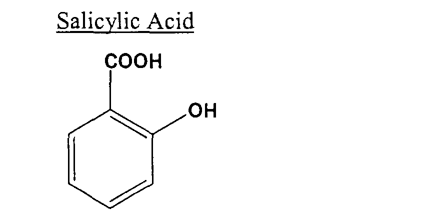

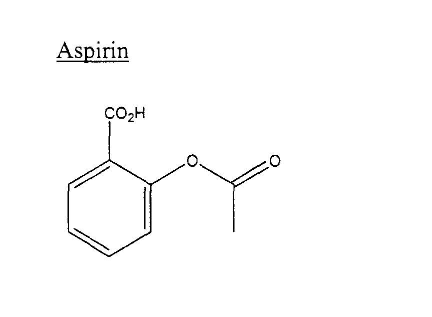

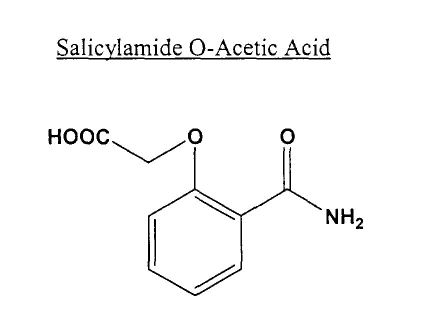

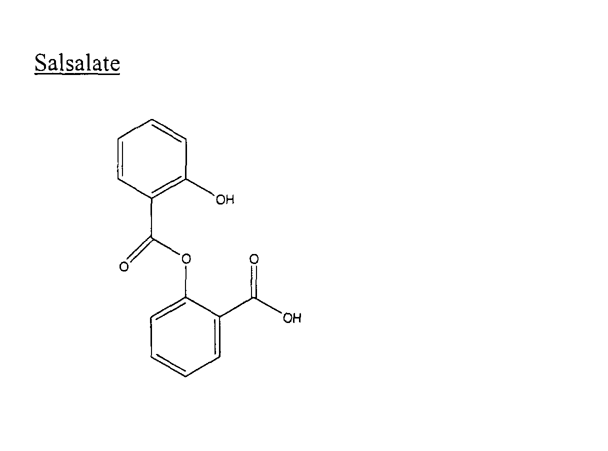

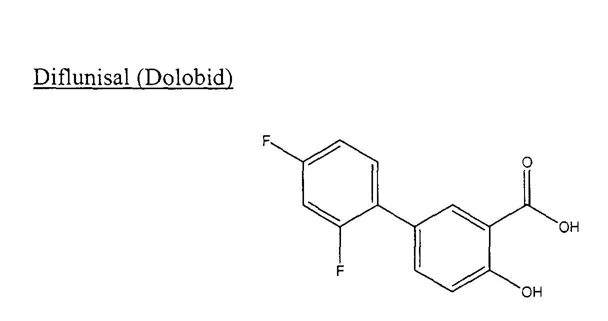

Arylacetic Acids: The prototype arylacetic acid is indomethacin. Suitable congeners of indimethacin include tolmetin, diclofenac, etodolac, lodrine, nabumetone, and 6-MNA. These compounds are represented by the following chemical structures.

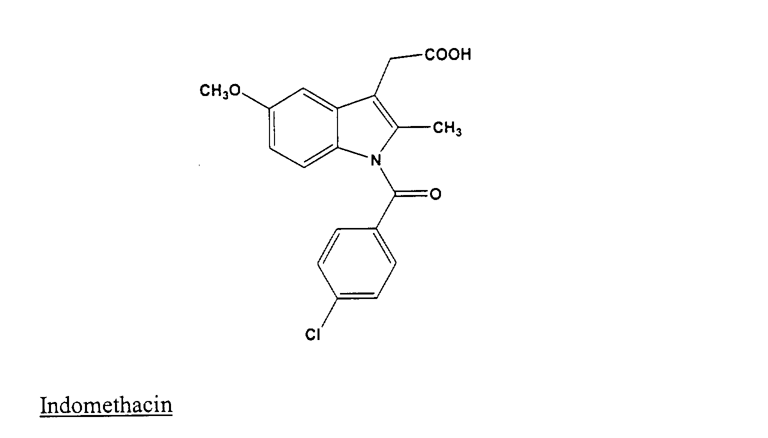

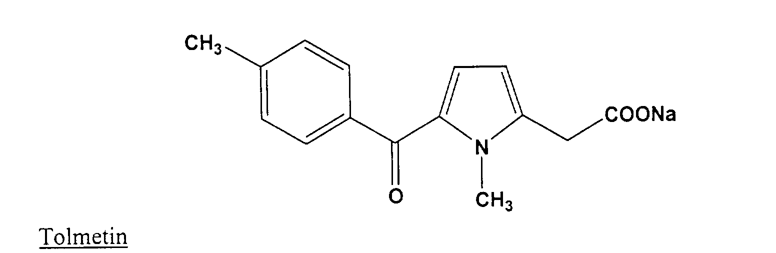

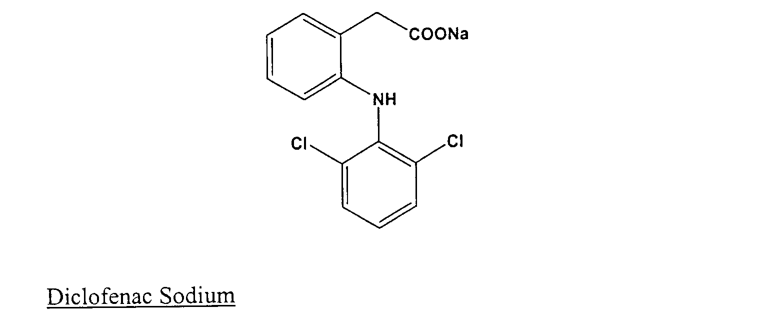

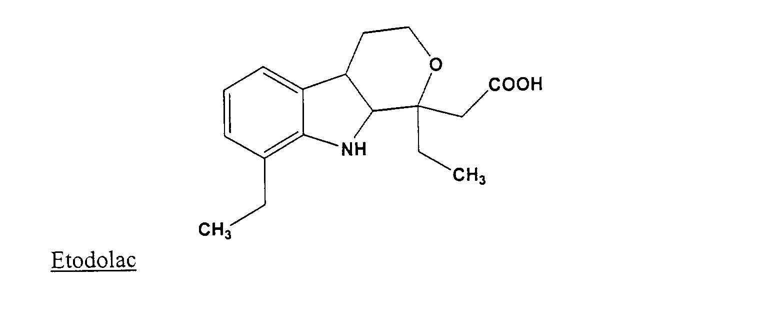

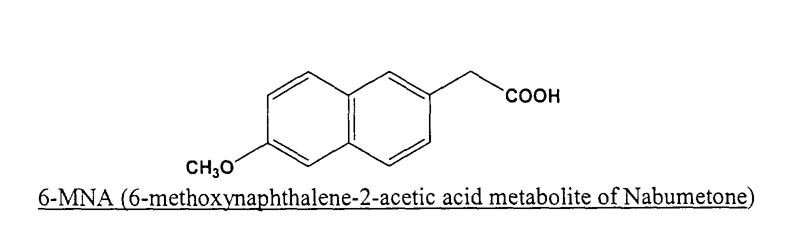

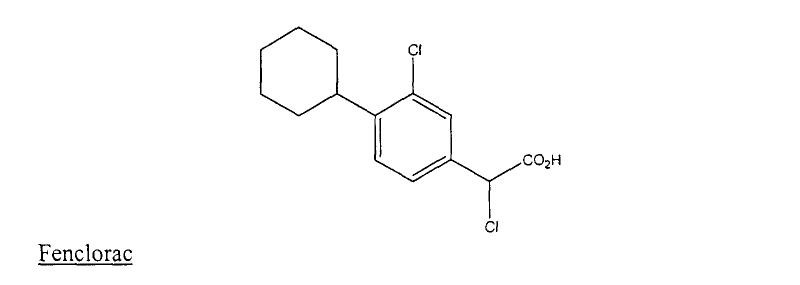

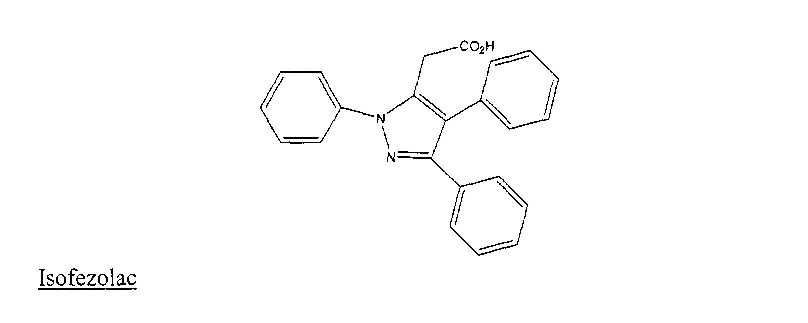

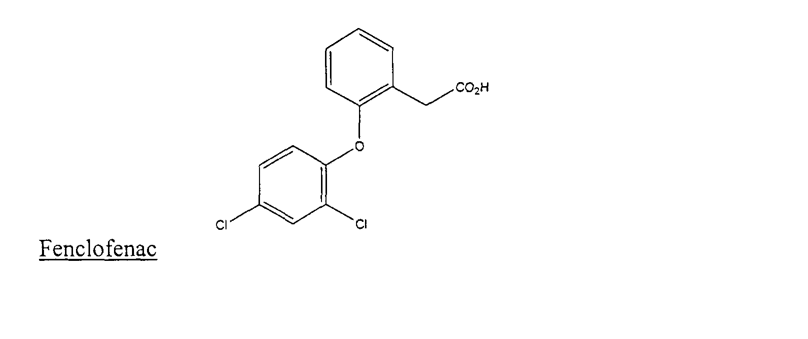

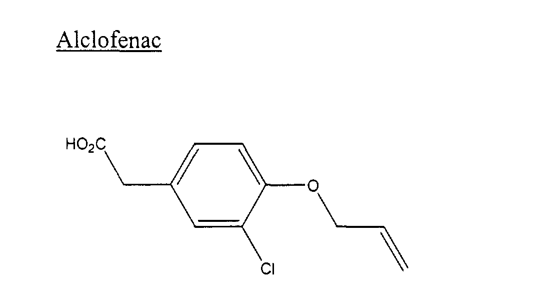

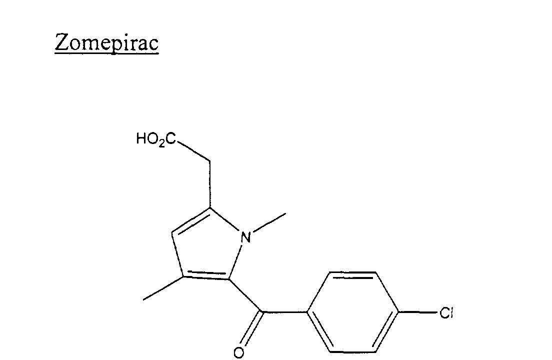

Arylpropionic Acids: Ibuprofen is the prototype drug in this class. Suitable congeners include naproxen, ketoprofen, fenoprofen, suprofen, flurbiprofen, ketorolac, carprofen, and oxaprozin. These compounds can be represented by the following chemical structures:

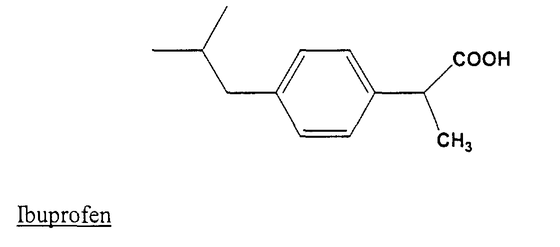

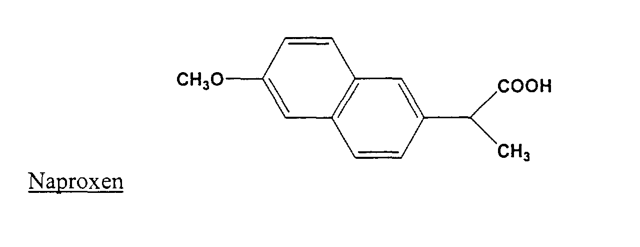

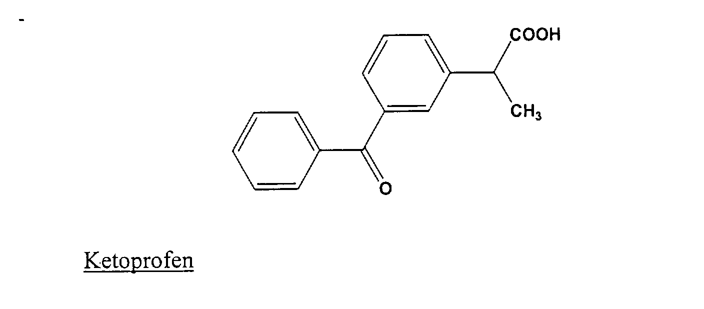

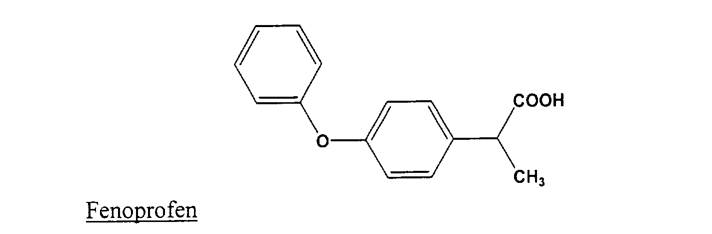

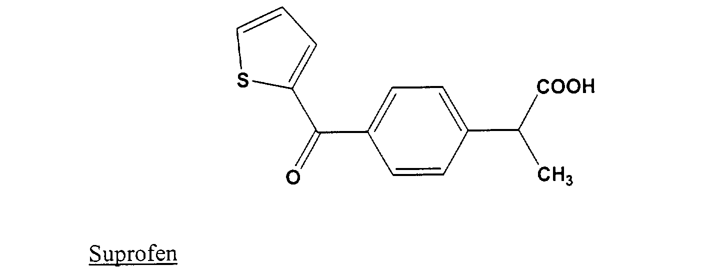

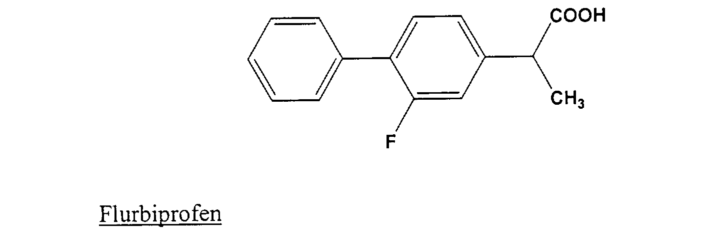

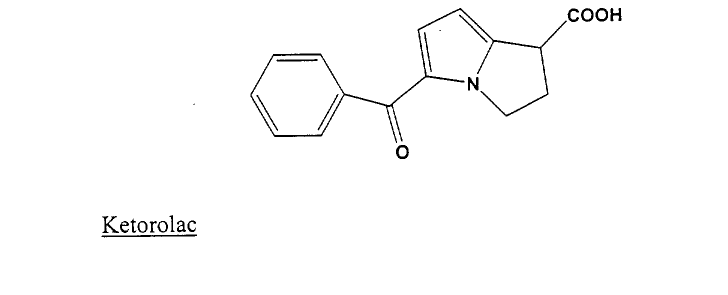

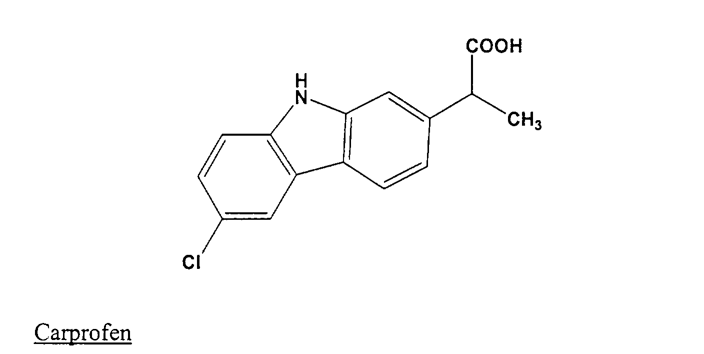

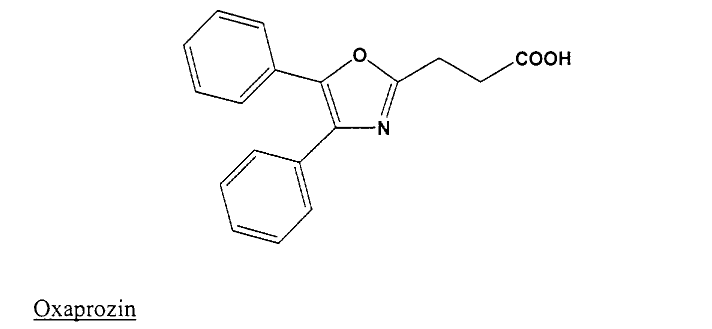

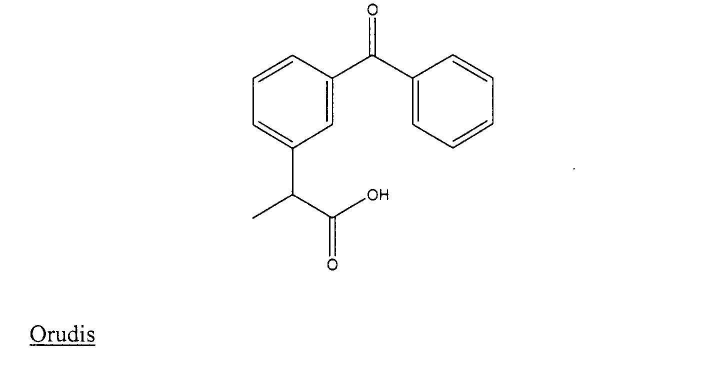

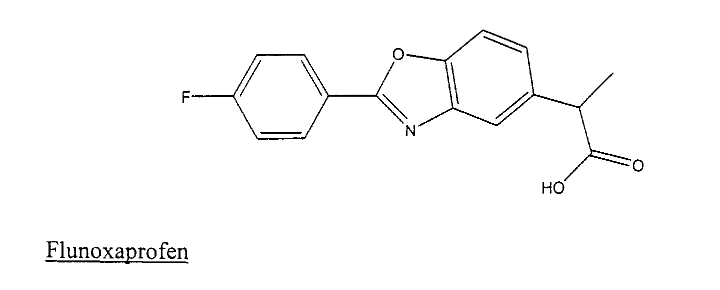

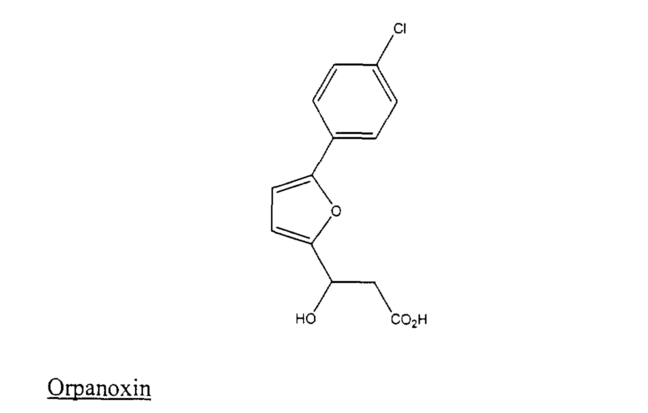

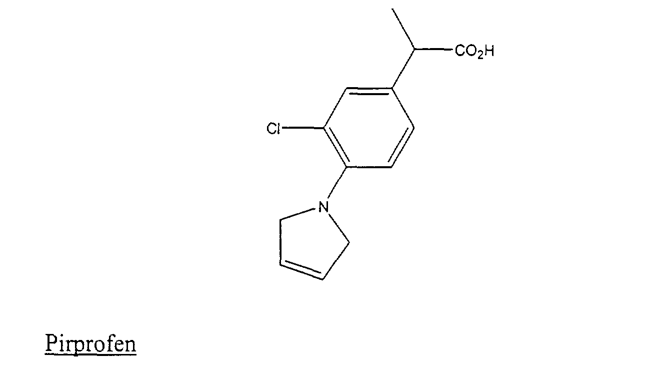

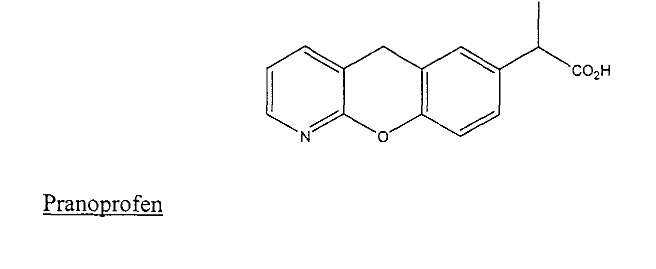

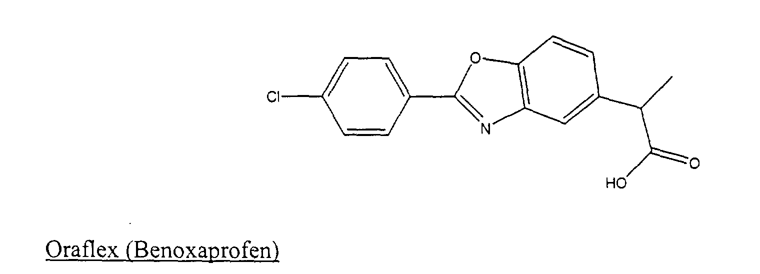

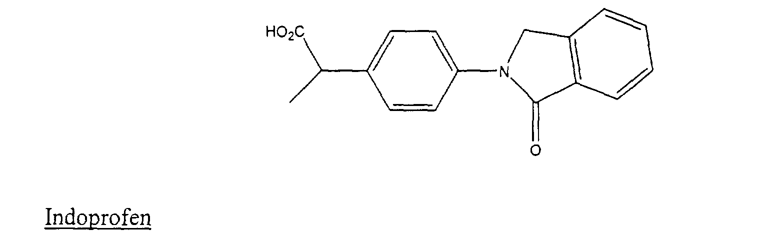

Fenamic Acids (N-Arylanthranilic Acids): The prototype drug in this class is mefenamic acid. A particularly suitable congener of mefenamic acid is meclofenamate. Mefenamic acid and meclofenamate can be represented by the chemical structures below:

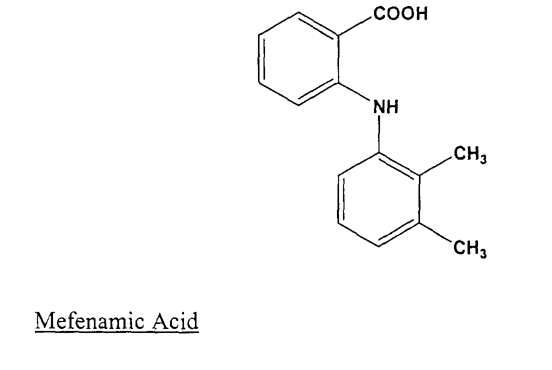

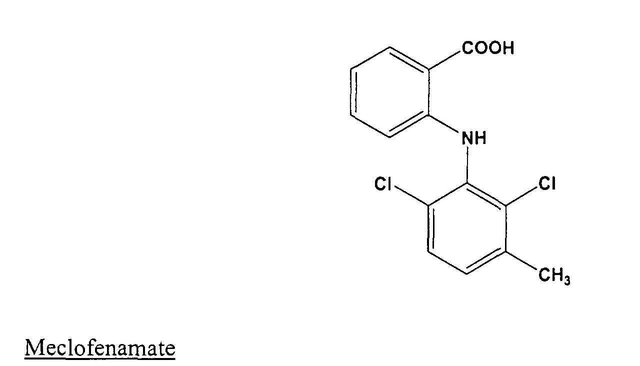

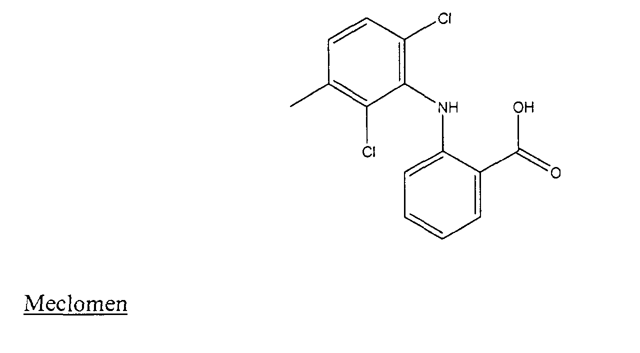

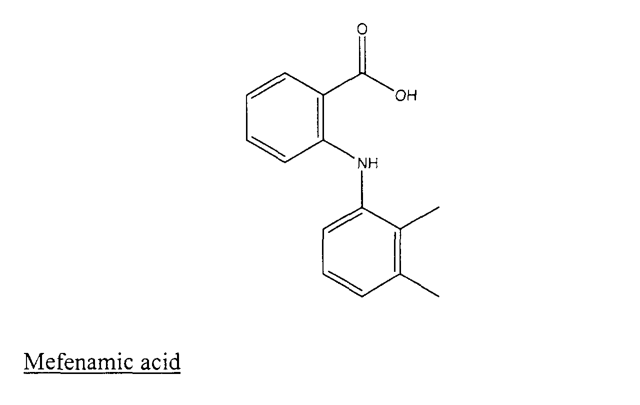

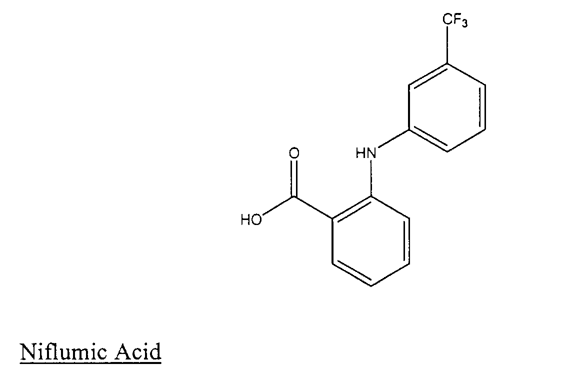

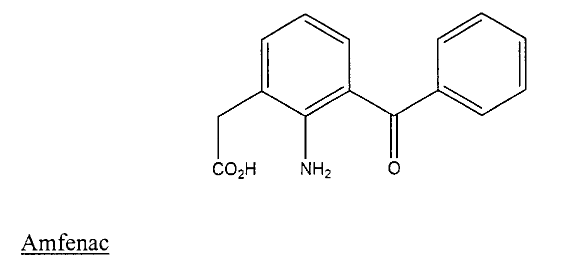

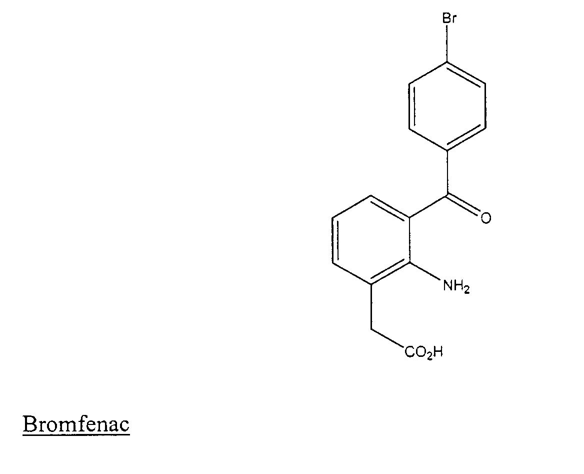

### Other Aryl Acids

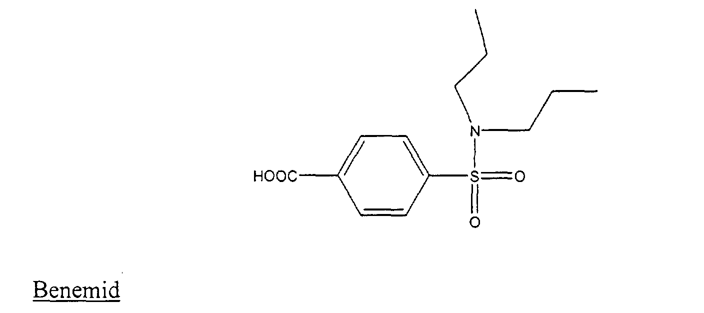

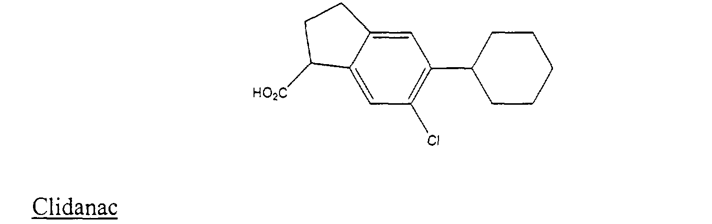

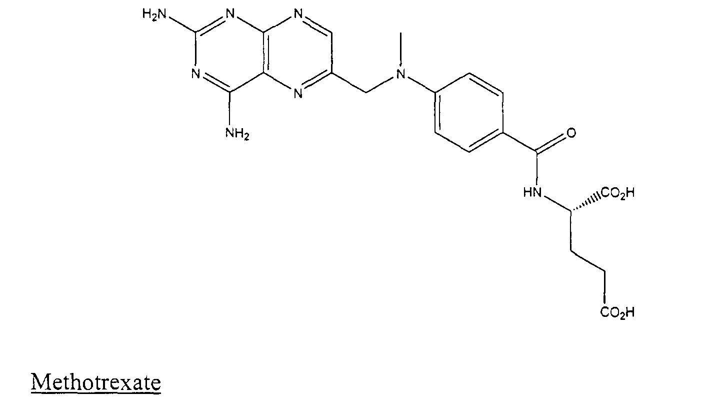

Other examples of NSAIDS that can be modified in accordance with the present invention include:
- tolfenamic acid: 2-{(3-chloro-2-methylphenyl)-amino}benzoic acid.
- fenclozic acid: 2-(4-chlorophenyl)-4-thiazoleacetic acid.
- fenbufen: 3-(4-biphenylcarbonyl)propionic acid.

As mentioned above, the invention can be practiced with any drug that possesses, in its active form, a carboxylate function. Moreover, the NSAIDs and other pharmaceutical agents of the present invention may have asymmetric centers and occur as racemates, racemic mixtures, individual diastereomers, or enantiomers, with all isomeric forms being included in the present invention. Dexketoprofen is an exemplary NSAID which is optically active, and which is encompassed within the general scope of this invention.

Other suitable parent compounds include a diverse array of suitable drugs, including muscle relaxants such as baclofen, diuretics such as ethacrynic acid, and antiepileptic drugs such as valproic acid. The chemical structures for baclofen, valproic acid, and ethacrynic acid are given below:

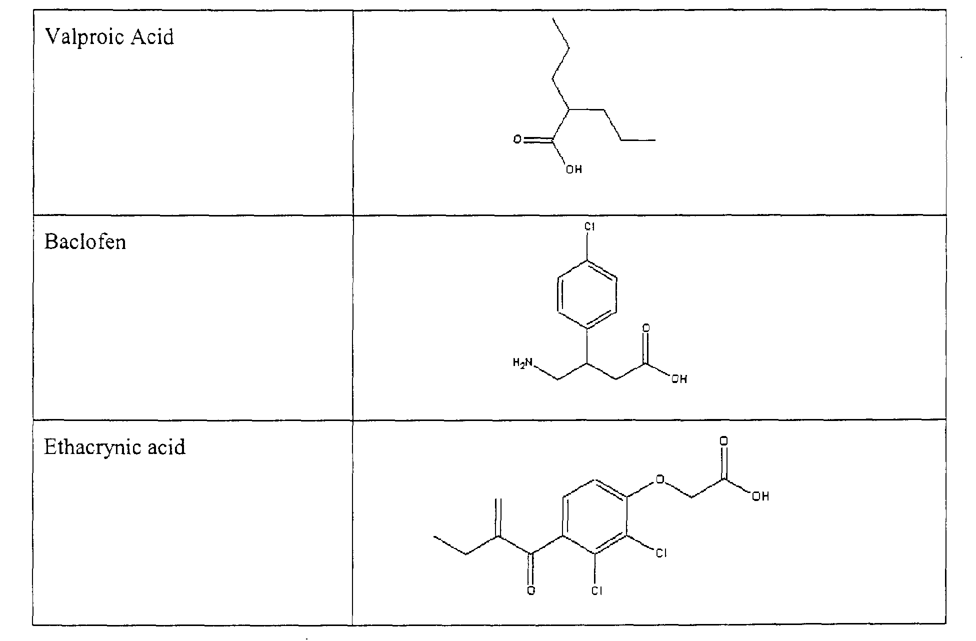

The following is a brief list of other classes of known therapeutic agents which can be linked to form prodrugs according to the present invention, and whose pharmacological profile in the metabolic system of animals is thereby greatly facilitated:
(1) amino acids
(2) depsipeptides
(3) peptides
(4) polypeptides
(5) proteins
(6) psychotropic medications known as
   (a) tranquilizers
   (b) sedatives
   (c) antidepressants
   (d) neuroleptics
   (e) hypnotics
   (f) muscle relaxants
   (g) anticonvulsants
   (h) analgesics
   (i) analeptics
   (j) anesthetics
   (k) antiParkinsonian agents
   (l) CNS stimulants
   (m) psychostimulants
(7) antiasthma compounds
(8) antispasmotics
(9) anorexics
(10) cardiovascular agents
   (a) antiarthymics
   (b) antihypertensives
   (c) cardiac glycosides
   (d) antidiuretics
   (e) antimigraines
   (f) antithrombotics
(11) antibacterials and antiseptics
(12) antibiotics
(13) antineoplastic drugs
(14) anticoagulants
(15) antidiabetic agents
(16) antidiarrheals
(17) antidotes
(18) antifungal agents
(19) antihistamines
(20) antiherpes (and other antiviral)
(21) antimetabolites
(22) antimalarials
(23) antiemetics
(24) antiparasitics
(25) antipruiritics
(26) antipyretics
(27) antispasmotics, anticholinergics
(28) biologicals
(29) bronchodilators
(30) calcium preparations
(31) antihyperlipidemics
(32) contraceptives
(33) cough and cold preparations
(34) decongestants
(35) dental preparations
(36) dermatologicals
(37) diagnostics
(38) dietary supplements
(39) hormones
(40) immunosuppressives
(41) ophthalmologicals
(42) parasympatholytics
(43) parasmypathomimetics
(44) prostaglandins

Antibiotics: The following are examples of antibiotics that contain a carboxylic acid moiety, and thus can be linked to the esterifying agent of the present invention through that functional moiety, using standard chemical reactions for covalent bond formation by derivatization of a carboxylic acid.

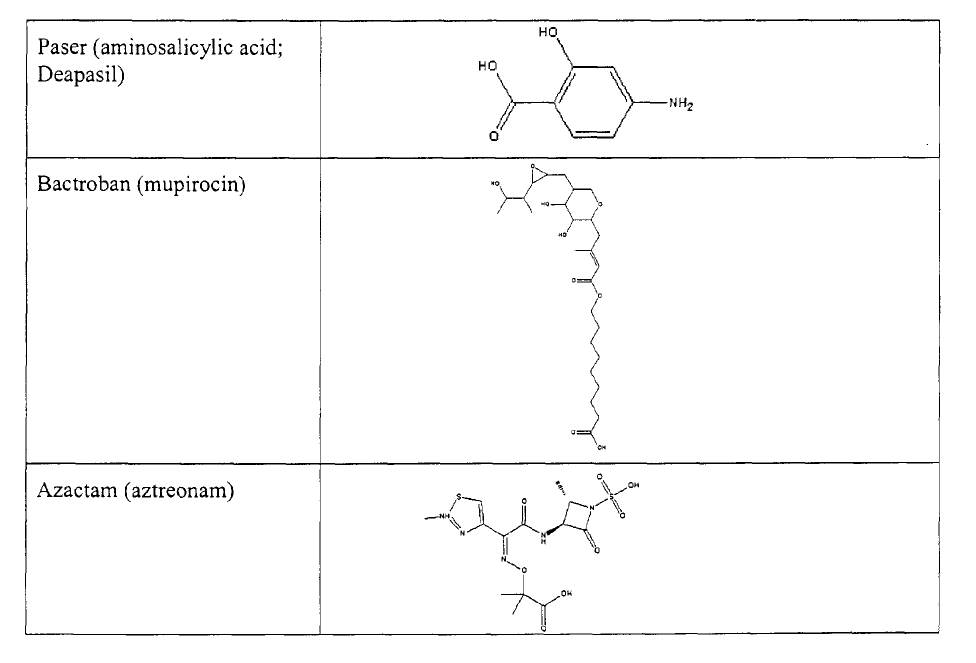

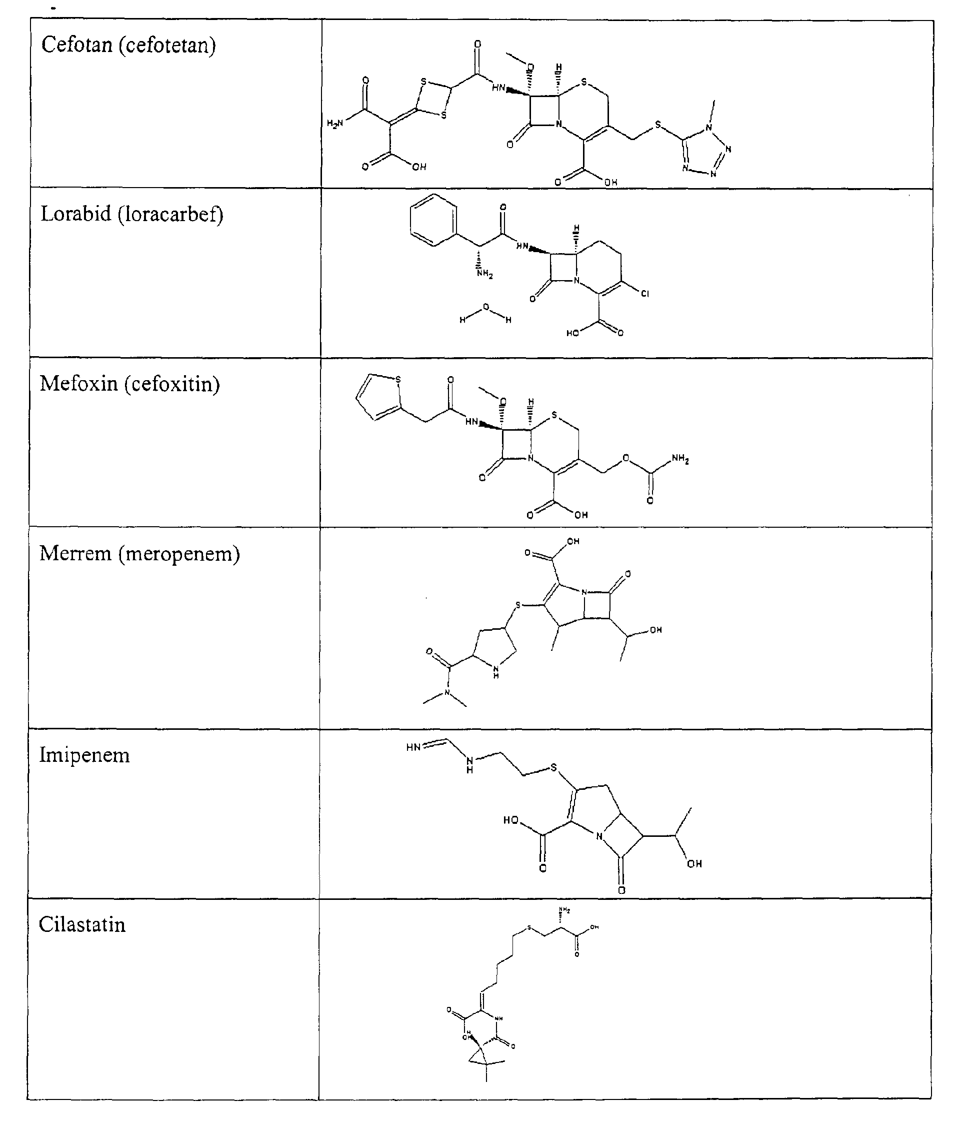

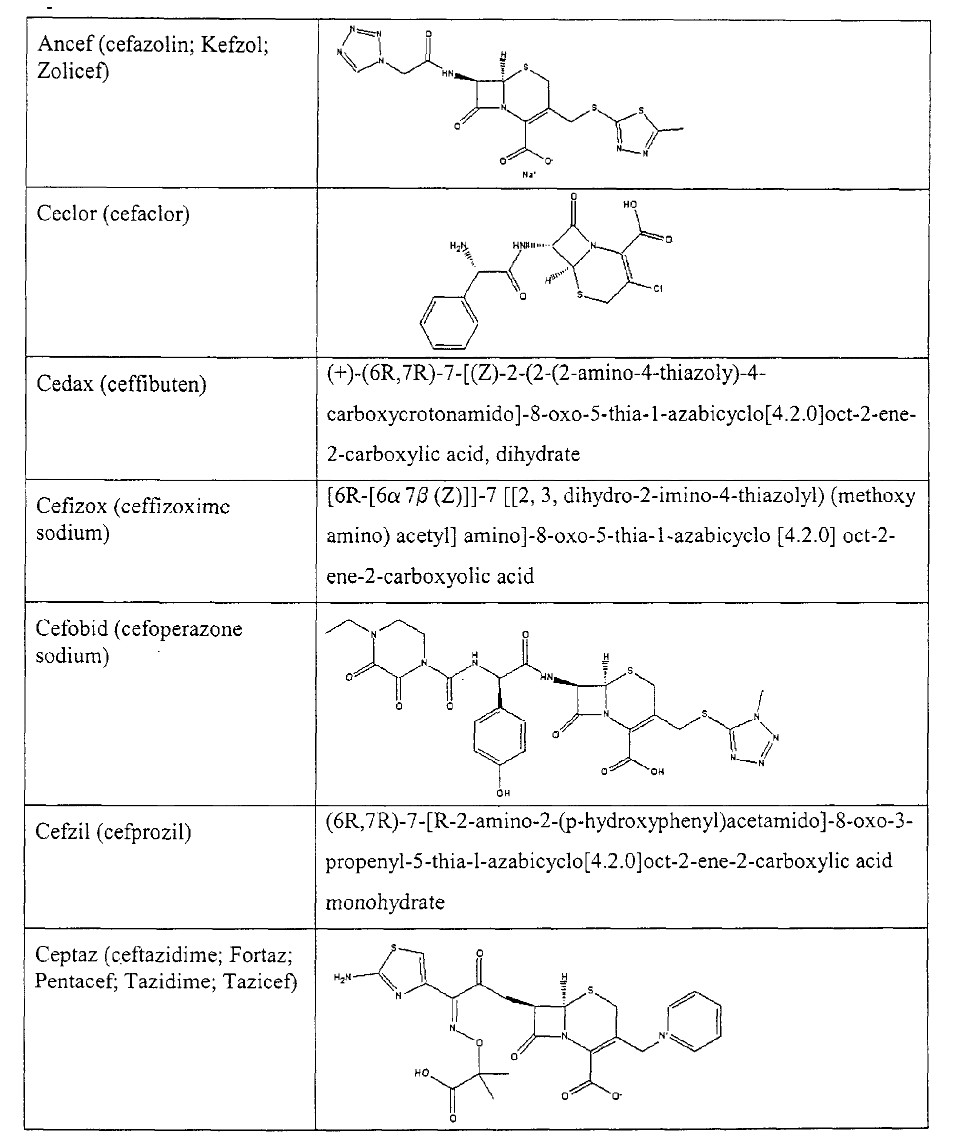

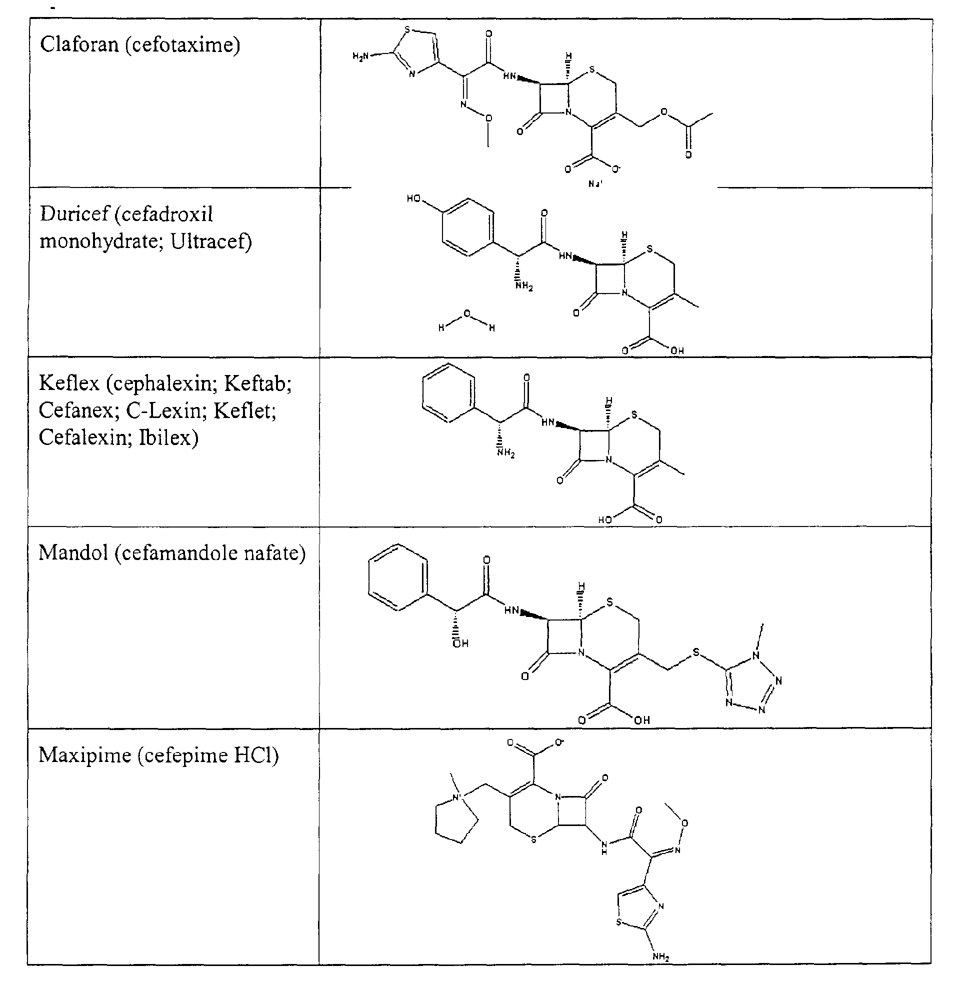

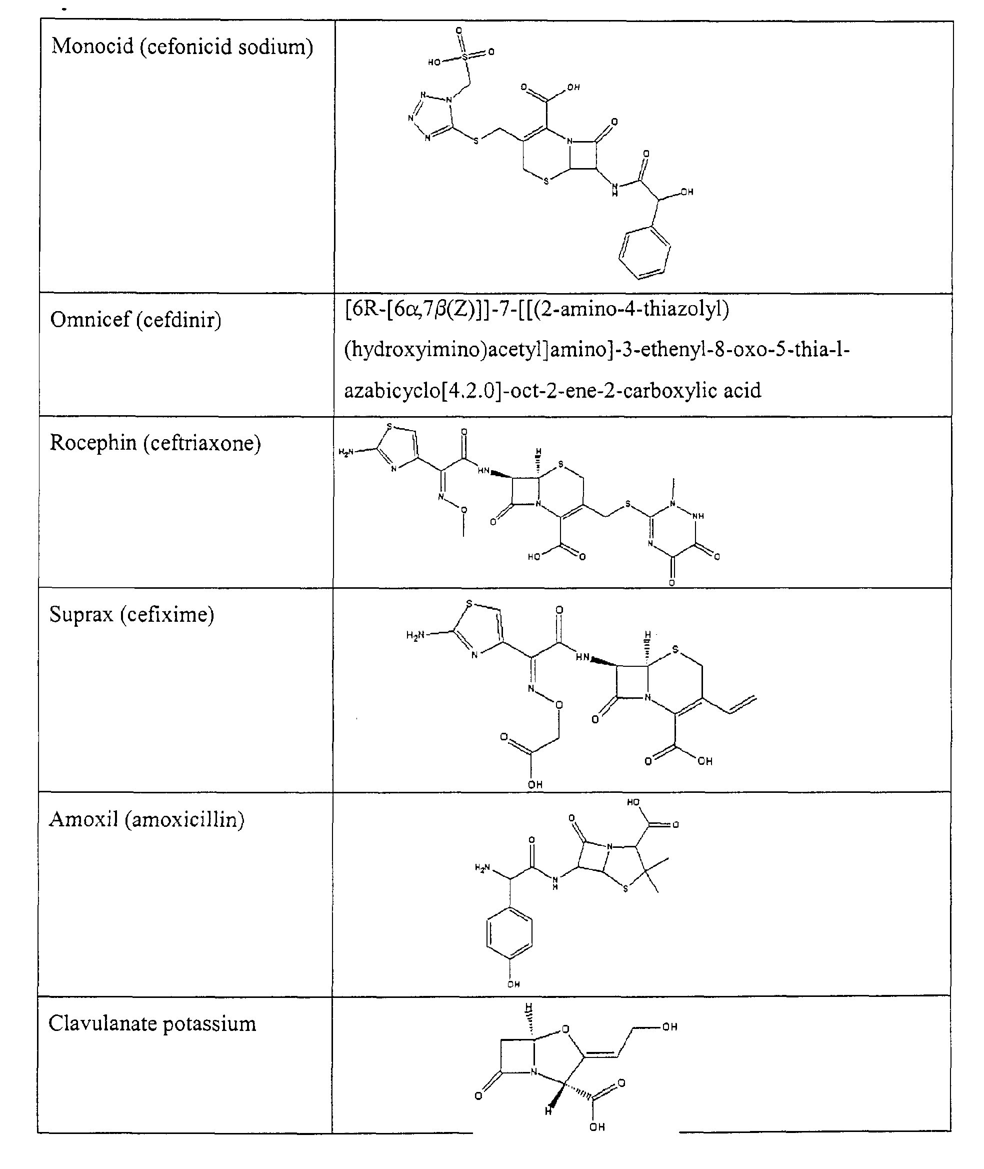

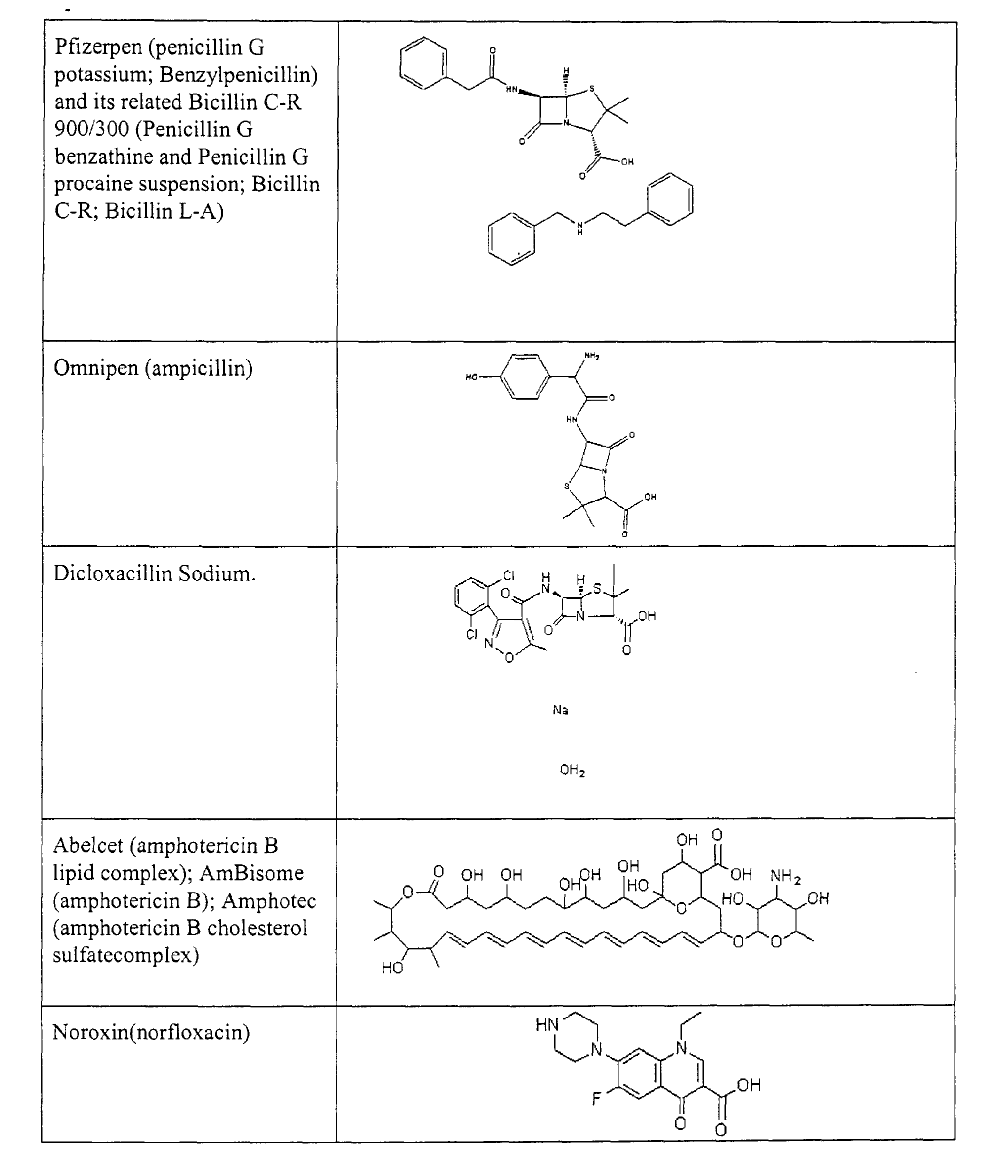

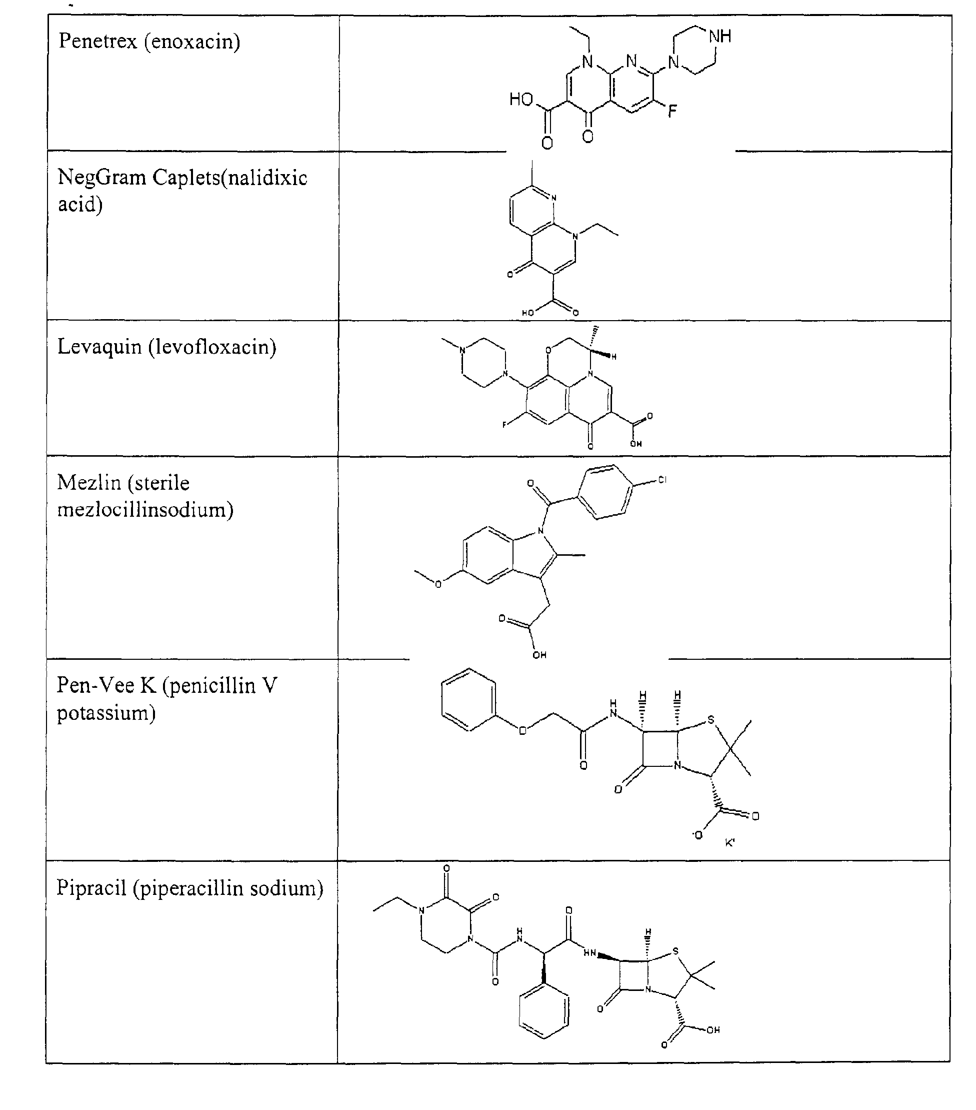

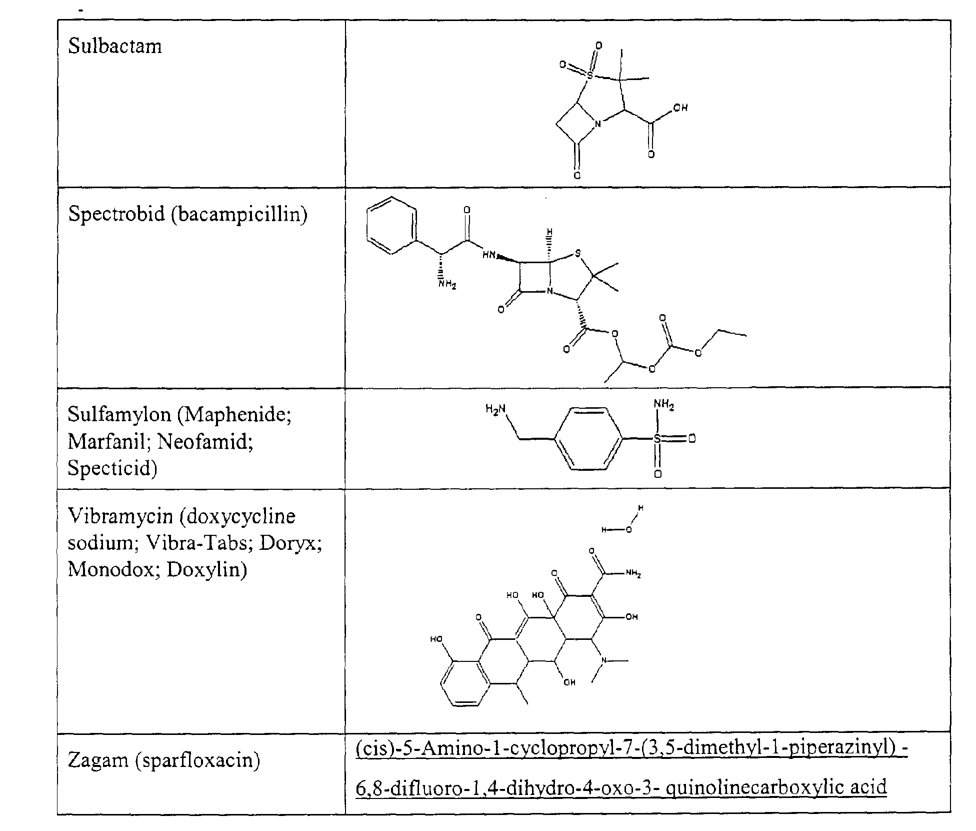

The following are examples of cardiovascular agents that contain a carboxylic acid moiety, and thus can be linked to the esterifying agent of the present invention through that functional moiety, using standard chemical reactions for covalent bond formation by derivatization of a carboxylic acid.

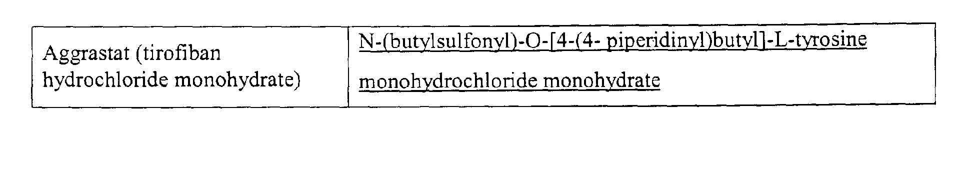

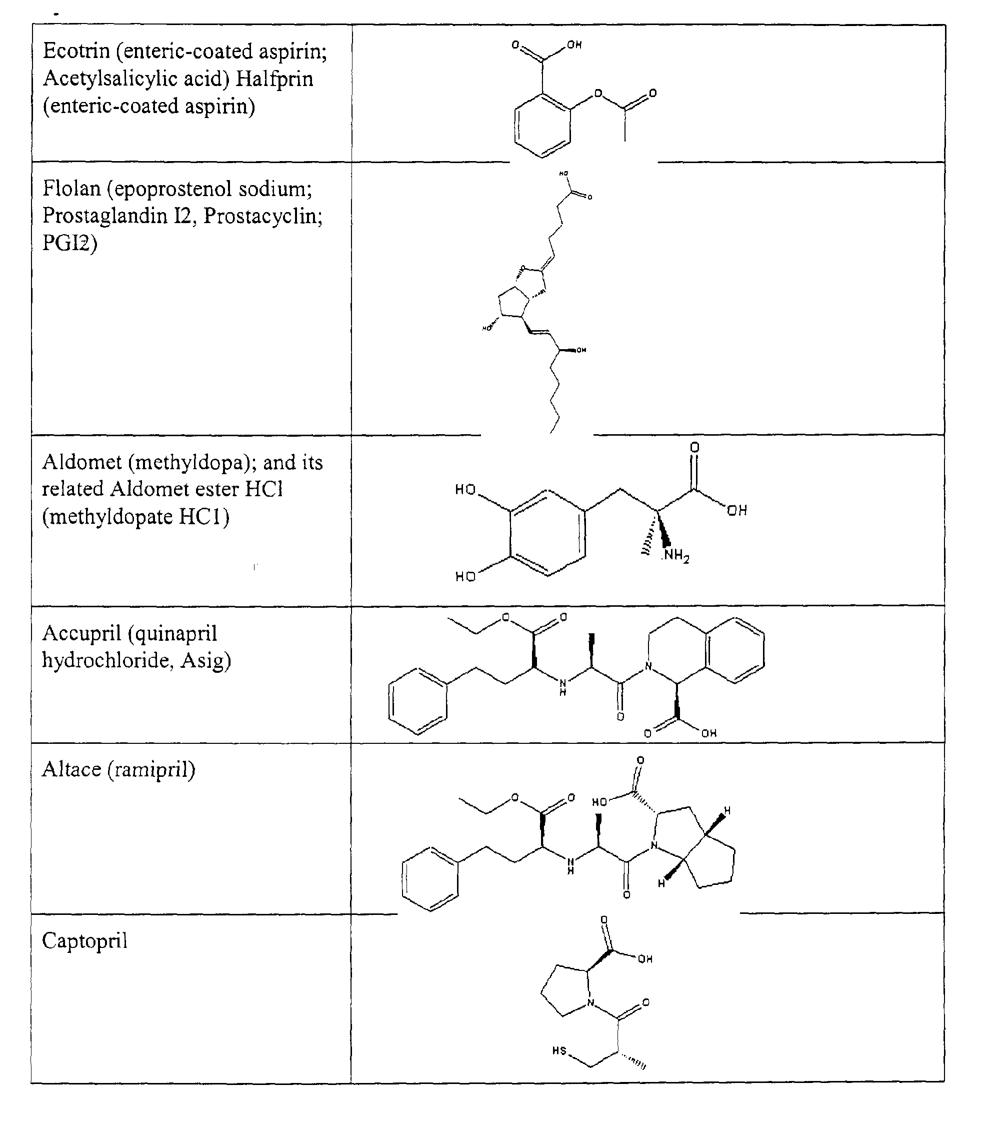

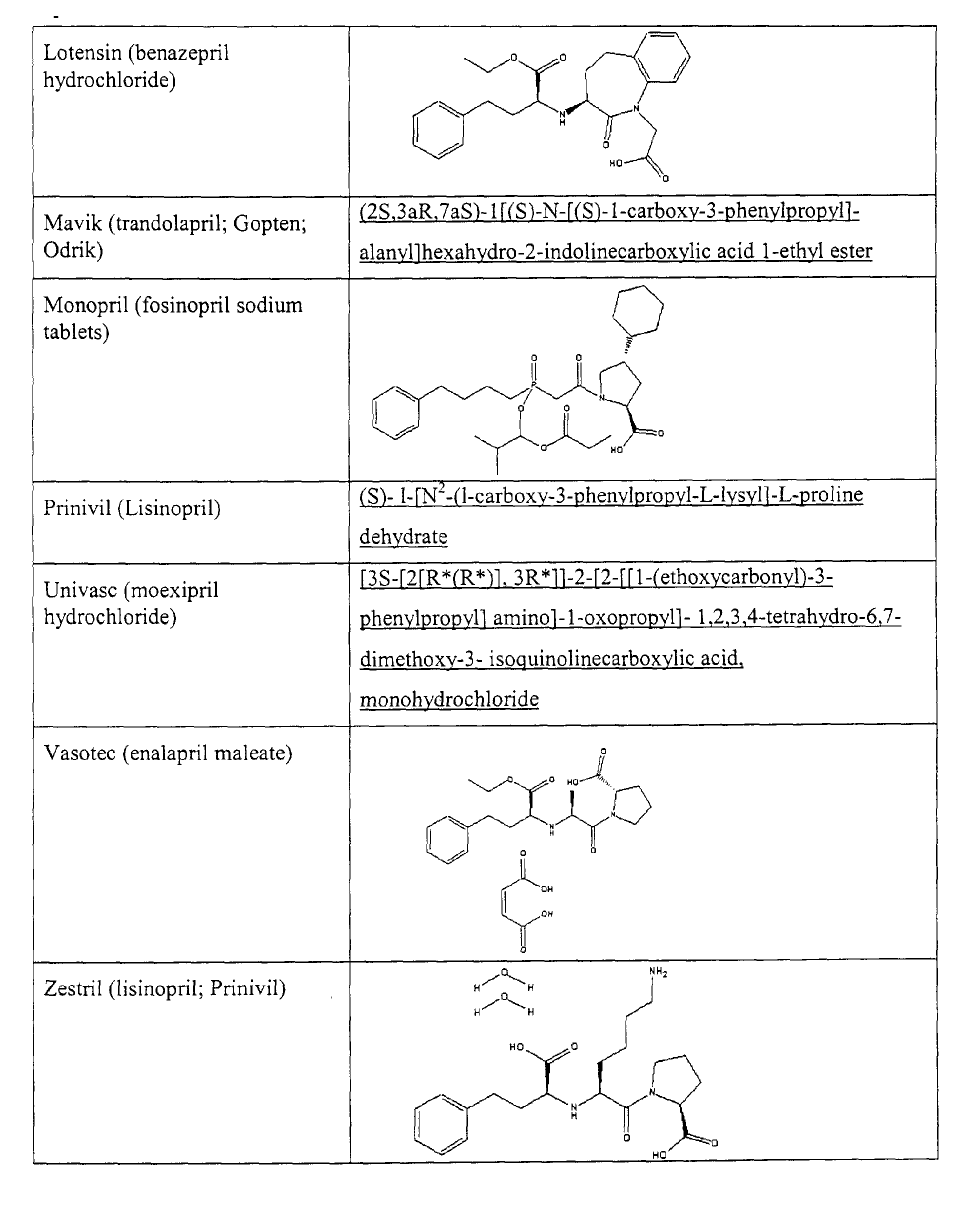

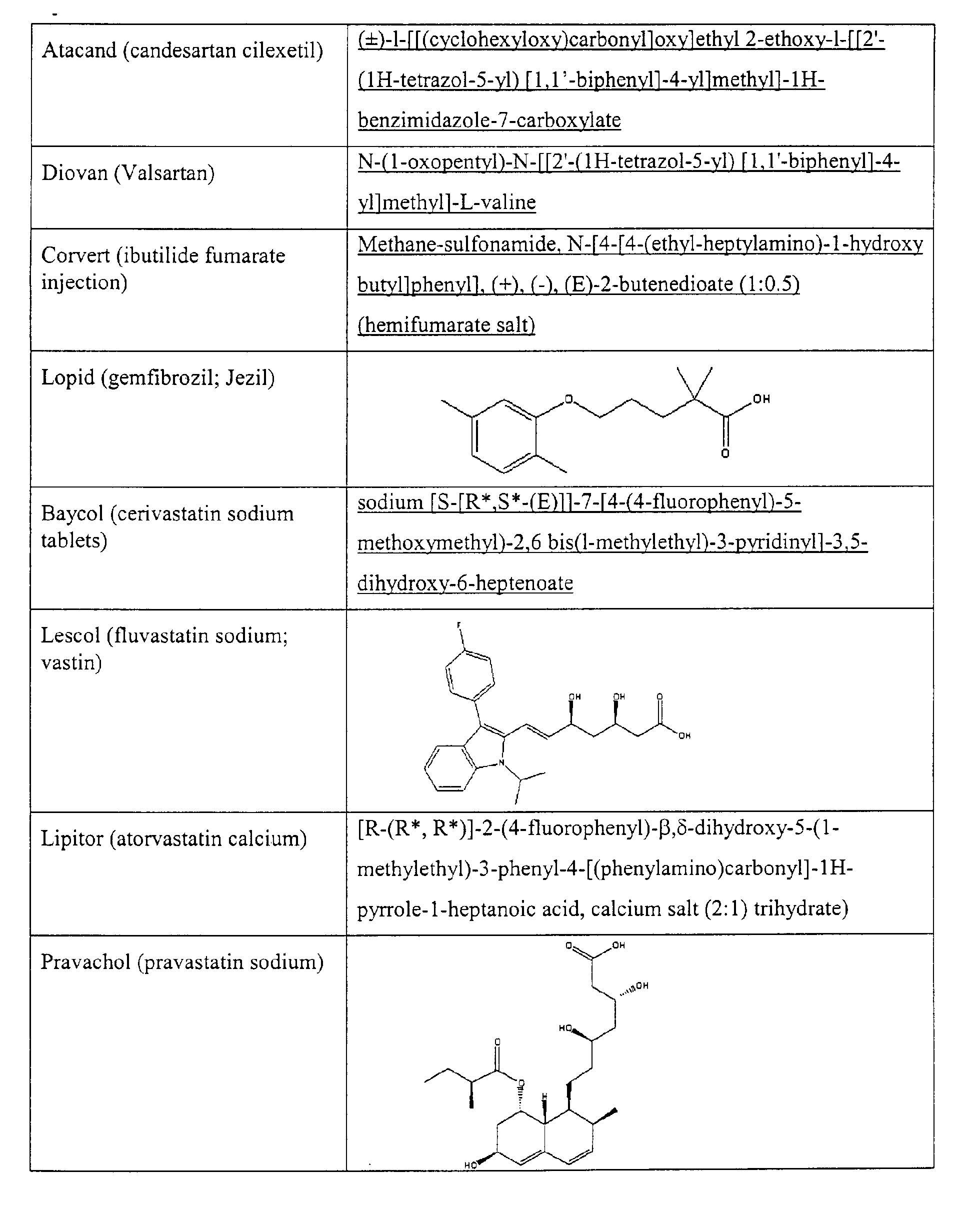

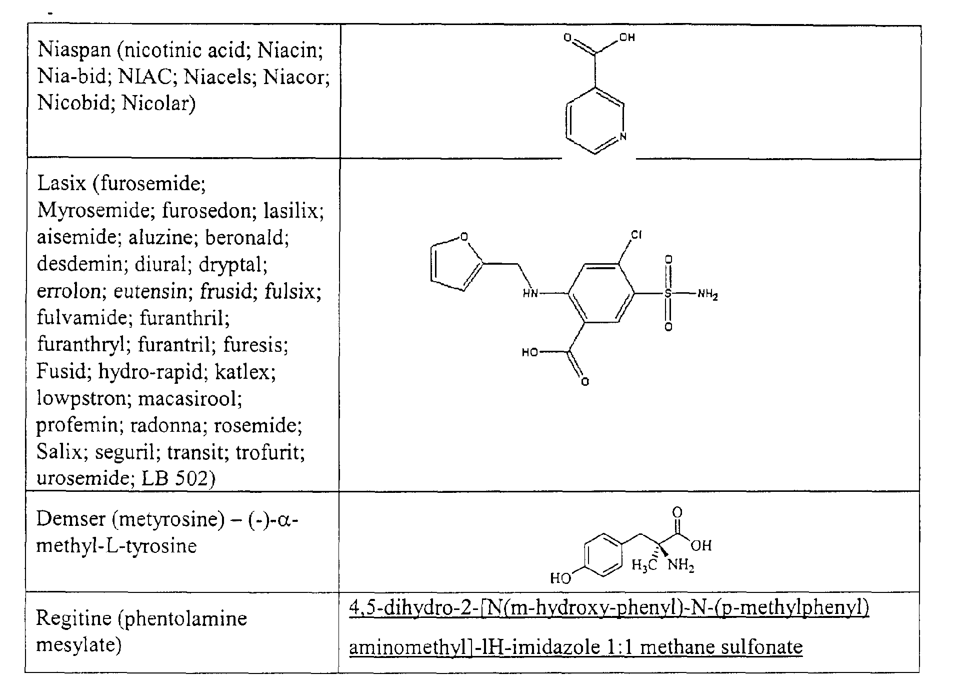

The following are examples of antiproliferative agents that contain a carboxylic acid moiety, and which can be linked to the esterifying agent of the present invention, using standard chemical reactions for covalent bond formation by derivation of a carboxylic acid function.

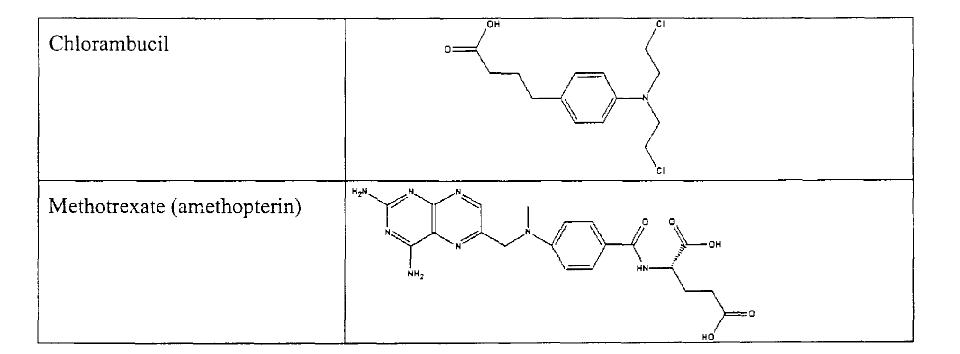

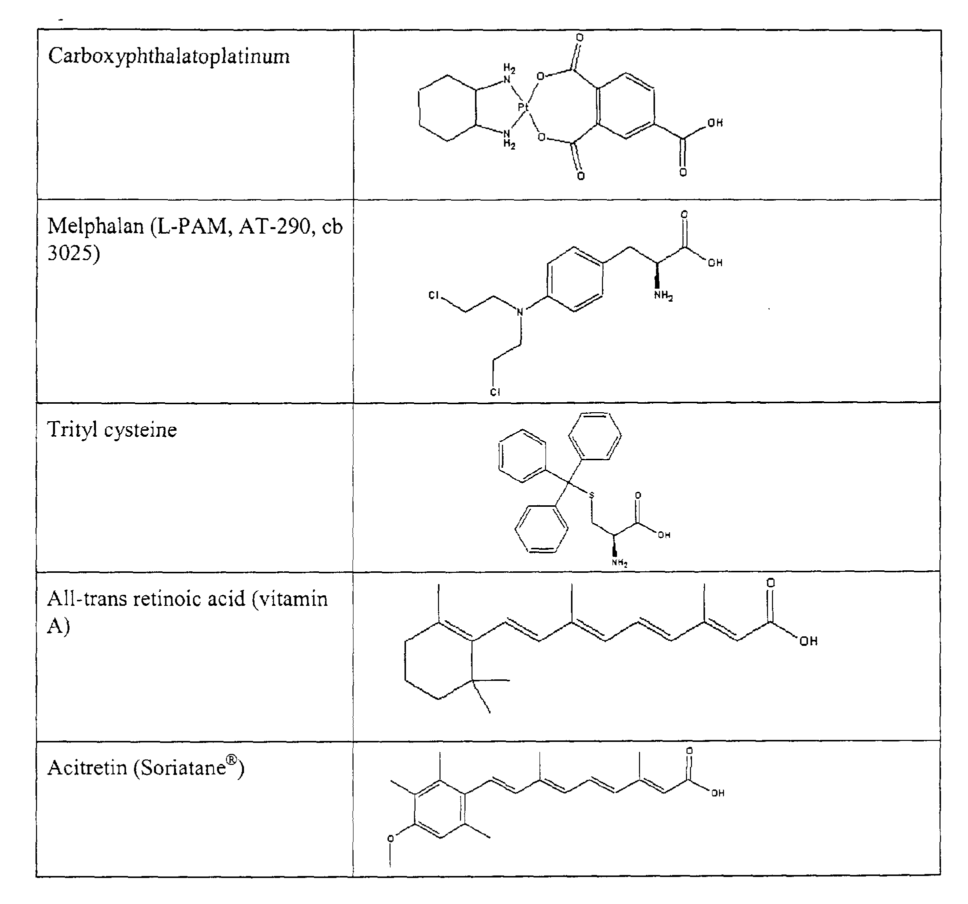

### Synthetic Methods

Numerous methods are available and known in the art for making esters of carboxylic acids, which can be utilized to prepare a prodrug of a selected carboxylic acid containing biologically active compound. In a preferred embodiment the novel prodrugs of the present invention are prepared in a simple one-step reaction by reacting the parent drug of formula RCOOH, or a salt thereof, with an esterifying compound of formula X-spacer-OC(O)R', yielding compounds of the general formula RC(O)O-spacer-OC(O)R', wherein:
a. RC(O)- is the acyl residue of a pharmaceutically active agent,
b. Spacer is Cₙ alkyl,
c. n is 1, 2, 3, 4, 5, or 6,
d. R' is substituted or unsubstituted heteroaryl or heterocycle, and
e. X is a leaving group.

According to the invention, the reaction is preferably performed in the presence of a polar solvent such as dimethylformamide or acetone. In the compounds of the above formula I, X is preferably Cl or Br or tosylate. X is most preferably bromo however, because bromine provides a significantly faster rate of esterification that with other halogen derivatives. The fact that the esterification with the bromo derivative proceeds under relatively mild conditions is of particular significance when preparing prodrugs of NSAIDs in the free carboxylic acid form, because of the sensitivity of many of these drugs to even mild reaction conditions.

Depending upon the functional groups that are present on the NSAID, the prodrugs can also be prepared by condensation or coupling reactions that are generally known to those skilled in the art. Condensation can be achieved with RC(O)OH, with an alcohol of formula HO-spacer-OC(O)R', yielding compounds of the general formula RC(O)O-spacer-OC(O)R', wherein:
a. RC(O)- is the acyl residue of a pharmaceutically active agent,
b. Spacer is Cₙ alkyl,
c. n is 1, 2, 3, 4, 5, or 6, and
d. R' is substituted or unsubstituted heteroaryl or heterocycle.

In these reactions, the condensation can be achieved, optionally in the presence of an acid, with the appropriate alcohol. Alternatively, the condensation can be achieved with the aid of a coupling reagent. Possible coupling reagents are any reagents that promote coupling, including but not limiting to, Mitsunobu reagents (e.g. diisopropyl azodicarboxylate and diethyl azodicarboxylate) with triphenylphosphine or various carbodiimides.

The carboxylic acids or salts of NSAIDs and other suitable drugs are reacted with the novel esterifying compounds such as N-[(2-haloethyloxy)carbonyl]morpholine to produce the novel prodrug esters. Preferred classes of esterifying compounds include N-[(2-haloethyloxy)carbonyl] morpholines, and more generally compoounds of the formula: 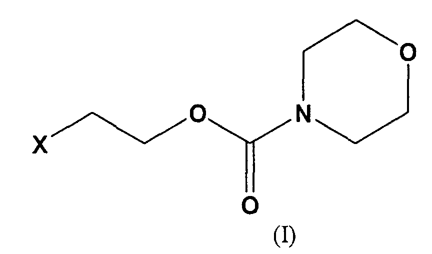 wherein X is a leaving group, and is preferably Cl or Br or tosylate. The esterifying compound N-[(2-haloethyloxy)carbonyl]morpholine can be prepared by reacting morpholine with 2-haloethylchloroformate, which is a known chemical intermediate, in the presence of benzene or toluene as a solvent and a suitable base such as pyridine or sodium hydroxide.

### EXAMPLES

### Example 1 -- Synthesis of N-[(2-bromoethyloxy)carbonyl]morpholine

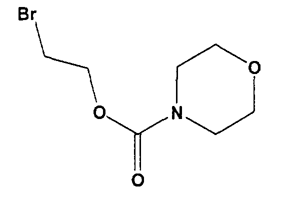

A mixture of 100 g morpholine and 200 ml of benzene containing 90.1 gm of pyfidine were mixed. To this mixture, 215 gm of 2-bromoethylchloroformate was added. The reaction mixture was refluxed for 8 hours. Solids were removed by filtration. The solution was evaporated, yielding an oily material. Distillation under vacuum yielded a pure oily material which solidified on cooling.
Melting Point: 42-44°C.
IR(cm⁻¹): Carbonyl at 1700
¹H NMR,(CDC13), δ 3.4 (m,4H,CH2-N-CH2); 3.6(t,2H, CH2Br, J= 6 Hz), 3.7 (m,4H,CH2-O-CH2, 4.4 (t,2H,CH2OCO, J=6 Hz).

### Example 2 -- Preparation of 1-(p-chlorobenzoyl)-5-methoxy-2-methylindole-3-acetic acid morpholinocarbonyloxyethyl ester

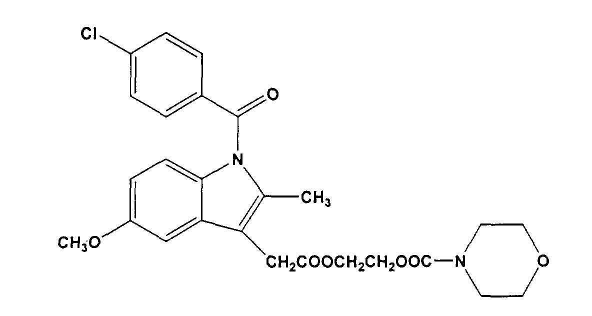

Anhydrous N-[(2-bromoethyloxy)carbonyl]morpholine (1.9 gm) was added to a solution of 1-(p-chlorobenzoyl)-5-methoxy-2-methylindole-3-acetic acid sodium salt (Indomethacin sodium) (3 gm) in 40 ml methanol. The mixture was heated for 20 hours at 60° C. The methanol evaporated under vacuum. The reaction was cooled to room temperature and 20 ml ethyl acetate was added to the reaction mixture, which was then filtered and washed twice with 25 ml water. The organic layer was then dried over anhydrous MgSO₄. A pure oily product was obtained after evaporation of ethyl acetate which was solidified on cooling. Recrystallization from methanol gave the indomethacin ester.
Melting point: 85-86° C.
IR cm^{-1:} Carbonyls at 1732, 1706, 1670.
¹H NMR,(CDC13), δ 2.3 (s,3H, vinyl CH3), 3.7 (s,2H,CH2CO), 3.2-3.5
(m,8H, morpholine), 3.9 (s,3H, OCH3), 4.3 (s(distorted),4H, OCH2CH2O), 6.6-7.8 (m,7H, aromatic).

### Example 3 - Preparation of (+)-6-Methoxy-a-methyl-2-naphthaleneacetic acid morpholinocarbonycarbonyloxyethyl ester

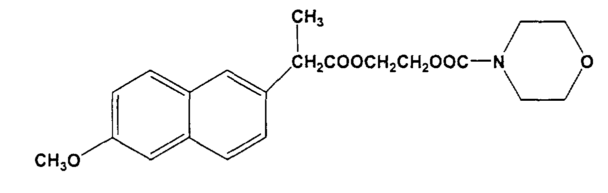

Anhydrous N-[(2-bromoethyloxy)carbonyl]morpholine (3 gm) was added to a solution of (+)-6-Methoxy-a-methyl-2-naphthaleneacetic acid sodium salt (naproxen sodium) (3 gm) in 20 ml dimethylformamide. The mixture was stirred for 48 hours at room temperature. The methanol was then evaporated under vacuum, and the reaction product cooled to room temperature. 20 ml ethyl acetate was added to the reaction mixture, and then filtered. The filtrate was washed twice with 25 ml water. The organic layer dried over anhydrous MgSO4. A pure oily product was obtained after evaporation of ethyl acetate which was solidified on cooling. Recrystallization from aqueous methanol gave naproxen ester.
Melting point⁼ 69-70° C
IR cm^{-1:} Carbonyls at 1732, 1706.
1H NMR,(CDC13), δ 1.6 (d,3H, a-CH3, J=7 Hz), 3.1-3.7(m, 8H,morpholine),3.8 (q, 1H, benzylic ), 3.9 (s,3H, OCH3), 4.2-4.4 (m,4H,OCH2CH2O), 7.1-7.8 (m,6H, aromatic).

### Example 4 - Preparation of 2-[(2,6-Dichlorophenyl])amino]benzene-acetic acid morpholinocarbonyloxyethyl ester

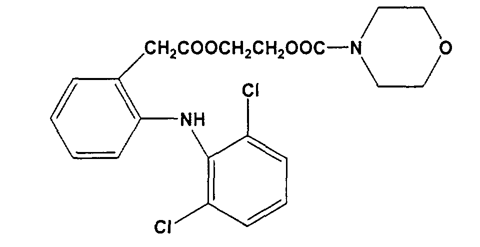

Anhydrous N-[(2-bromoethyloxy)carbonyl]morpholine (2.25 gm) was added to a solution of 2-[(2,6-Dichlorophenyl])amino]benzene-acetic acid sodium salt (Diclofenac sodium) (3 gm) in 5 ml dimethylformamide. The mixture was stirred for 48 hours at room temperature. 20 ml ethyl acetate was added to the reaction mixture, then filtered and washed twice with 25 ml water. The organic layer dried over anhydrous MgSO₄. A pure oily product was obtained after evaporation of the ethyl acetate which was solidified on standing. The product was recrystalized from methanol to give diclofenac ester.
Melting point: 49-51 °C.
IR cm^{4:} carbonyls at 1732, 1706.
¹H NMR,(CDC13), δ 3.2-3.7 (m,8H, morpholine); 3.85 (s,2H,benzylic -CH2-);
4.25-4.40 (m,4H,-O-CH₂ -CH₂ O-); 6.9-7.4 (m,7H, aromatic).

### Example 5 -- Preparation of m-Benzoylhydratropic acid morpholinocarbonyloxyethyl ester

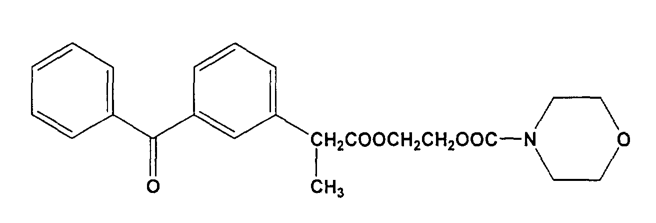

Anhydrous N-[(2-bromoethyloxy)carbonyl]morpholine (2.6 gm) was added to a solution of m-benzoylhydratropic acid sodium salt(ketoprofen) (3 gm) in 5 ml dimethylformamide. The mixture was stirred for 20 hours at 60°C. 20 ml ethyl acetate was added to the reaction mixture, and then filtered. The filtrate was washed twice with 25 ml water, and the organic layer dried over anhydrous MgSO₄. A pure oily product (Ketoprofen-ester) was obtained after evaporation of the ethyl acetate.
Oil at room temperature.
IR cm^{-1:} Carbonyls at 1732, 1706, 1670.
NMR, ¹H NMR,(CDC13), δ 1.4 (d, 3H,α-CH3, J=7 Hz), 3.1-3.7 ( m, 8H, morpholine ), 3.8 ( q, 1H, benzylic, J= 7 Hz), 4.2-4.4 (m, 4H, OCH2CH20), 7.2-7.9 (m, 9H, aromatic).

### Example 6 - Preparation of 2-[(2,3-Dimethylphenyl)amino]-benzoic acid morpholinocarbonyloxyethyl ester

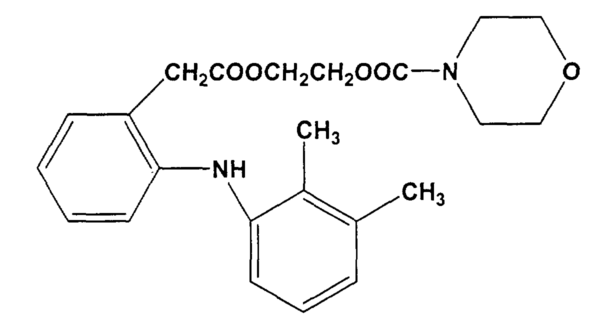

Using the same procedure as that given in example 4, the prodrug of mefenamic acid was produced.
Oil at room temperature.
IR cm⁻¹:Carbonyls at 1732, 1706
¹H NMR,(CDCI3), δ 2.2 (s,3H, CH₃), 2.3 (s, 3H, CH₃),3.4-3.6 (m, 8H, morpholine), 4.3-4.5(m, 4H, -OCH2CH20-), 6.7-7.9(m, 7H, aromatic), 9.2(s, 1H, NH).

### Example 7 - Preparation of α-Methyl-4-(2-methylpropyl)benzene-acetic acid morpholinocarbonyloxy ethyl ester

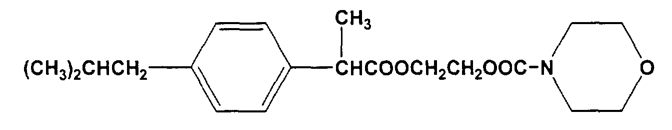

Using the same procedure as that given in example 4, the prodrug of ibuprofen was produced.
Oil at room temperature.
IR cm⁻¹: Carbonyls at 1732, 1706
1H NMR,(CDC13), δ 0.9 (d,6H,CH(CH3)2), 1.4 (d,3H, δ-CH3),1.8 (m, 1H,CH3-CH-CH3),2.4 (2H,d,bezylic CH2),3.8 (d,1H,CHCO, J=7Hz), 3.3-3.8 (m,8H, morpholine),4.3 (m,4H,O(CH2)20),7.1 (d,2H, aromatic,J=8Hz),7.2 (d,2H,aromatic,J=8Hz)

### Example 8 -- 5-benzoyl-2,3-dihydro- 1H-Pyrrolizine- 1-carboxylic acid morpholinocarbonyloxyethyl ester

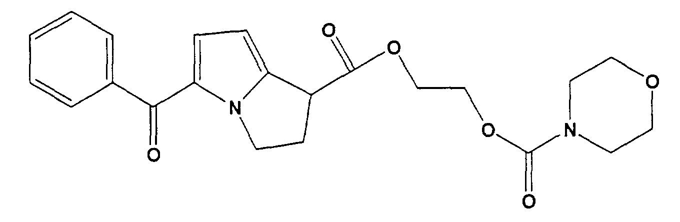

Using the same procedure as that given in example 4, the prodrug of Ketorolac was produced.
M.P.= 69-72°C.
IR cm^{-1:} carbonyls at 1745, 1705, 1625.

1H NMR,(CDC13), δ 2.8 (m,2H, pyrrolidine); 3.3-3.7 (m,8H, morpholine); 4.1 (m, 1H, pyrrolidine); 4.3-4.7 (m,6H, O(CH2)20 & -CH2 pyrrolidine); 6.1 (d, 1H, pyrrol H, J=4Hz); 6.8 (d, 1H, pyrolle H, J= 4 Hz); 7.4-7.6 (m,3H, aromatic ); 7.8 (d,2H, aromatic, J= 7 Hz).

### Pharmaceutical Formulations

The prodrugs described herein can be administered in any effective amount known for the particular indication for which the parent compound is prescribed. The prodrugs can be administered by any appropriate route, including orally, topically, ophthalmically, parenterally, or intravenously, in liquid or solid form. A suitable dosage can be determined by one skilled in the art by taking into consideration (1) the dosing requirements of the parent drug, and (2) the *in vivo* release profile of the prodrug.

The concentration of active compound in the drug composition will depend on absorption, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

A preferred mode of administration of the active compound is oral. Oral compositions will generally include an inert diluent or an edible carrier. They may be enclosed in gelatin capsules or compressed into tablets. For the purpose of oral therapeutic administration, the active compound can be incorporated with excipients and used in the Form of tablets, troches, or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents.

The compound can be administered as a component of an elixir, suspension, syrup, wafer, chewing gum or the like. A syrup may contain, in addition to the active compounds, sucrose as a sweetening agent and certain preservatives, dyes and colorings and flavors.

The compound or a pharmaceutically acceptable prodrug or salts thereof can also be mixed with other active materials that do not impair the desired action, or with materials that supplement the desired action, such as antibiotics, antifungals, anti-inflammatories, or other antivirals, including other nucleoside compounds. Solutions or suspensions used for parenteral, intradermal, subcutaneous, topical, or ophthalmic application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parental preparation can be enclosed in ampoules, disposable syringes or multiple dose vials made of glass or plastic.

If administered intravenously, preferred carriers are physiological saline or phosphate buffered saline (PBS).

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation.

Liposomal suspensions (including liposomes targeted to infected cells with monoclonal antibodies to viral antigens) are also preferred as pharmaceutically acceptable carriers. These may be prepared according to methods known to those skilled in the art, for example, as described in U.S. Patent No. 4,522,811 (which is incorporated herein by reference in its entirety). For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound or its monophosphate, diphosphate, and/or triphosphate derivatives is then introduced into the container. The container is then swirled by hand to free lipid material from the sides of the container and to disperse lipid aggregates, thereby forming the liposomal suspension.

Topical and ophthalmic formulations and preparations may conveniently be presented as a solution, an aqueous or oily suspension, or an emulsion. The active ingredient may also be presented as a bolus, electuary or paste. While the carrier substance used in a particular topical composition is not critical to this invention, in a preferred embodiment the carrier fluid of a topical formulation as disclosed herein comprises water and a thickening agent.

Preferred thickening agents include cellulose or a chemically treated derivative of cellulose. Derivatives of cellulose which have been chemically treated to make them more hydrophilic (such as hydroxyethyl and hydroxymethyl derivatives, which have numerous additional hydroxy groups bonded to the starting cellulose molecules) have been widely used as thickening agents in gels that are applied to the skin. Other suitable thickening agents include acacia, agar, alginate, carrageenan, gum tragacanth, xanthan gum, collagen, carboxypolymethylene, glyceryl monostearate, polyvinylpyrrolidone, and polyacrylamide. The thickening agents listed above are relatively inactive biologically, and basically serve as carrier substances.

Other components, including preservatives (such as chlorhexidine gluconate), anti-crystallization agents (such as glucono-delta-lactate), fragrances, coloring agents, alkaline or acidic or buffering agents to maintain the proper pH, and soothing or anti-swelling agents (such as lanolin, aloe vera extract, or hydrocortisone) can be added to the compositions described herein.

The therapeutic compound is optionally administered topically by the use of a transdermal therapeutic system (see, Barry, Dermatological Formulations, (1983) p. 181 and literature cited therein). While such topical delivery systems have been designed largely for transdermal administration of low molecular weight drugs, by definition they are capable of percutaneous delivery. They can be readily adapted to administration of the therapeutic compounds of the invention by appropriate selection of the rate-controlling microporous membrane. Topical application can also be achieved by applying the compound of interest, in a cream, lotion, ointment, or oil based carrier, directly to the skin. Typically, the concentration of therapeutic compound in a cream, lotion, or oil is 1-2%.

For drug targeting to lung tissue, the therapeutic compound is formulated into a solution, suspension, aerosol or particulate dispersion appropriate for application to the pulmonary system. The therapeutic agent may be inhaled via nebulizer, inhalation capsule, inhalation aerosol, nasal solution, intratracheal as a solution via syringe, or endotracheal tube as an aerosol or via as a nebulizer solution. Aersols are prepared using an aqueous aerosol, liposomal preparation or solid particles containing the compound. A nonaqueous (e.g. fluorocarbon propellant) suspension could be used. Sonic nebulizers are preferred because they minimize exposing the therapeutic compound to shear, which can result in degradation of the compound.

The composition herein is also suitably administered by sustained release systems. The sustained release systems can be tailored for administration according to any one of the proposed administration regimes. Slow or extended-release delivery systems, including any of a number of biopolymers (biological-based systems), systems employing liposomes, and polymeric delivery systems, can be utilized with the compositions described herein to provide a continuous or long term source of therapeutic compound.

Suitable examples of sustained release compositions include semipermeable polymer matrices in the form of shaped articles, e.g., films, microcapsules, or microspheres. Sustained release matrices include, for example, polylactides (U.S. Pat. No. 3,773,919), copolymers of L-glutamic acid and γ-ethyl-L-glutamate (Sidman *et al.,* Biopolymers 22:547-556, 1983), orpoly-D-(-)-3-hydroxybutyric acid (EP 133,988). Sustained release compositions also include one or more liposomally entrapped compounds of formula I. Such compositions are prepared by methods known per se, e.g., as taught by Epstein *et al.* Proc. Natl. Acad. Sci. USA 82:3688-3692, 1985. Ordinarily, the liposomes are of the small (200-800 Å) unilamellar type in which the lipid content is greater than about 30 mol % cholesterol, the selected proportion being adjusted for the optimal therapy.

A variety of techniques to produce microparticles have been described in the prior art. For example, United Kingdom Patent Application No. 2,234,896 to Bodmer *et al.* describes a method of forming microparticles by mixing a solution of the polymer dissolved in an appropriate solvent with a solution of a drug. Microparticle formation is then induced by the addition of a phase inducing agent. European Patent Application 0 330 180 to Hyon *et al.* describes a process for preparing polylactic acid-type microparticles by adding a solution of a drug and a polymer in a mixed solvent to a phase inducing agent and evaporating the original solvent microparticle formation. Other examples of processes for preparing microparticles by phase separation technique have been described in U.S. Pat. Nos. 4,732,763 to Beck *et al.* and 4,897,268 to Tice *et al.* and by Ruiz *et al.* in the International Journal of Pharmaceutics (1989) 49:69-77 and in Pharmaceutical Research (1990) 9:928-934.

Throughout this application, various publications are referenced. The disclosures of these publications in their entireties are hereby incorporated by reference into this application in order to more fully describe the state of the art to which this invention pertains.

It will be apparent to those skilled in the art that various modifications and variations can be made in the present invention without departing from the scope or spirit of the invention. Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. A compound of the formula: RC(O)O-spacer-OC(O)R', or a pharmaceutically acceptable salt thereof, wherein:
a) RC(O)- is the acyl residue of an NSAID or other pharmaceutically active agent bearing a carboxylic acid function,
b) spacer is Cₙ alkyl,
c) n is from 1 to 6, and
d) R' is substituted or unsubstituted heteroaryl or heterocycle.

2. The compound of claim 1 wherein RC(O)- is the acyl residue of an NSAID.

3. The compound of claim 1 wherein spacer is -(CH₂)ₙ-.

4. The compound of claim 1 wherein spacer is ethylene.

5. The compound of claim 1 wherein R' is NR¹R², wherein R¹ and R² are C₂₋₄ alkyl or heteroalkyl that join to form a 5-7 membered heterocyclic or heteroaromatic ring, substituted or unsubstituted.

6. The compound of claim 1 wherein R' is NR¹R², and R¹ and R² combine to form morpholine.

7. The compound of claim 1 selected from
a) 1-(p-chlorobenzoyl)-5-methoxy-2-methylindole-3-acetic acid morpholinocarbonyloxyethyl ester;
b) (+)-6-methoxy-a-methyl-2-naphthaleneacetic acid morpholinocarbonycarbonyloxyethyl ester;
c) 2- [(2,6-dichlorophenyl])amino]benzene-acetic acid morpholinocarbonyloxyethyl ester;
d) m-benzoylhydratropic acid morpholinocarbonyloxyethyl ester;
e) 2-[(2,3-dimethylphenyl)amino]-benzoic acid morpholinocarbonyloxyethyl ester;
f) α-methyl-4-(2-methylpropyl)benzene-acetic acid morpholinocarbonyloxy ethyl ester; and
g) 5-benzoyl-2,3-dihydro- 1H-pyrrolizine- 1-carboxylic acid morpholinocarbonyloxyethyl ester.

8. The compound of claim 2 wherein the NSAID is:
a) a salicylate selected from aspirin, salicylamide O-acetic acid, salsalate, and diflunisal;
b) an arylacetic acid selected from indomethacin, tolmetin, diclofenac, etodolac, lodrine, nabumetone, 6-MNA, fenclorac, isofezolac, fenclofenac, alclofenac, and zomepirac;
c) an arylpropionic acid selected from ibuprofen, naproxen, ketoprofen, fenoprofen, suprofen, flurbiprofen, ketorolac, carprofen, oxaprozin, orudis, flunoxaprofen, orpanoxin, pirprofen, pranoprofen, oraflex, and indoprofen;
d) a fenamic acid selected from mefenamic acid, meclofenamate, meclomen, niflumic acid, amfenac, and bromfenac; or
e) benemid, clidanac, methotrexate, tolfenamic acid, fenclozic acid, or fenbufen.

9. The compound of claim 1 wherein RC(O)- is the acyl residue of a muscle relaxant, a diuretic, an antiepileptic, an antibiotic, a cardiovascular agent, or an antiproliferative agent.

10. A pharmaceutical composition comprising a compound of the formula: RC(O)O-spacer-OC(O)R' or a pharmaceutically acceptable salt thereof, and a pharmaceutically acceptable carrier, wherein:
a) RC(O)- is the acyl residue of an NSAID or other pharmaceutically active agent bearing a carboxylic acid function,
b) spacer is Cₙ alkyl,
c) n is from 1 to 6, and
d) R' is substituted or unsubstituted heteroaryl or heterocycle.

11. The pharmaceutical composition of claim 10, formulated for oral, topical, or ophthalmic delivery.

12. A compound of the formula X-spacer-OC(O)R', wherein:
a) X is a leaving group,
b) spacer is Cₙ alkyl,
c) n is 1-6, and
d) R' is substituted or unsubstituted heteroaryl or heterocycle.

13. The compound of claim 22 wherein X is bromine.

14. The compound of claim 22 which is N-[(2-bromoethyloxy)carbonyl] morpholine.

15. Use of a compound of the formula RC(O)O-spacer-OC(O)R', in the treatment of a disease wherein:
a) RC(O)- is the acyl residue of an NSAID or other pharmaceutically active agent for the disease bearing a carboxylic acid function,
b) spacer is Cₙ alkyl,
c) n is from 1 to 6, and
d) R' is substituted or unsubstituted heteroaryl or heterocycle.

16. The use of claim 15 wherein the disease is inflammation or an inflammatory disorder.

17. Use of a compound of the formula RC(O)O-spacer-OC(O)R', in the manufacture of a medicament for the treatment of a disease wherein:
a) RC(O)- is the acyl residue of an NSAID or other pharmaceutically active agent for the disease bearing a carboxylic acid function,
b) spacer is Cₙ alkyl,
c) n is from 1 to 6, and
d) R' is substituted or unsubstituted heteroaryl or heterocycle.

18. A method of making a prodrug comprising reacting a drug bearing a carboxylic acid function, or a salt thereof, with a compound of the formula X-spacer-OC(O)R', wherein:
a) X is a leaving group,
b) spacer is Cₙ alkyl,
c) n is 1-6, and
d) R' is substituted or unsubstituted heteroaryl or heterocycle.

19. A method of making a prodrug of the formula RC(O)O-spacer-OC(O)R', comprising condensing a drug of the formula RC(O)OH, or a salt thereof, with an alcohol of formula HO-spacer-OC(O)R', wherein:
a) RC(O)- is the acyl residue of a NSAID or other pharmaceutically active agent,
b) spacer is Cₙ alkyl,
c) n is from 1 to 6, and
d) R' is substituted or unsubstituted heteroaryl or heterocycle.
